# EUROPEAN PATENT SPECIFICATION

(11) **EP 1 347 048 B1**
(45) Date of publication and mention of the grant of the patent: **14.02.2007**
(21) Application number: 01271857.3
(22) Date of filing: 21.12.2001
(51) Int. Cl.: C12N 9/64, C12N 15/57, C07K 16/40, G01N 33/68

(54) **NOVEL PROTEASE**
NEUE PROTEASE
NOUVELLE PROTEASE

(30) Priority: 25.12.2000 JP 2000393372
(43) Date of publication of application: 24.09.2003
(73) Proprietor: Astellas Pharma Inc., Tokyo 103-8411 (JP)
(72) Inventor: YAMAJI, Noboru, c/o Astellas Pharma Inc., Chuo-ku, Tokyo 103-8411 (JP); NISHIMURA, Kouichi c/o Astellas Pharma Inc., Chuo-ku, Tokyo 103-8411 (JP); ABE, Kunitake, c/o Astellas Pharma Inc., Chuo-ku, Tokyo 103-8411 (JP); OGINO, Makoto, c/o Astellas Pharma Inc., Chuo-ku, Tokyo 103-8411 (JP)
(74) Representative: HOFFMANN EITLE
(86) International application number: PCT/JP2001/011251
(87) International publication number: WO 2002/051998

(56) References cited:
- WO-A-01/83782
- WO-A-02/31163
- WO-A-02/38744
- WO-A-03/027282
- WO-A-03/040393
- CLARK M E ET AL: "ADAMTS9, a novel member of the ADAM-TS/metallospondin gene family" GENOMICS, ACADEMIC PRESS, SAN DIEGO, US, vol. 67, no. 3, 1 August 2000 (2000-08-01), pages 343-350, XP002227221 ISSN: 0888-7543
- WANG LI ET AL: "Altered gene expression in kidneys of mice with 2,8-dihydroxyadenine nephrolithiasis" KIDNEY INTERNATIONAL, vol. 58, no. 2, August 2000 (2000-08), pages 528-536, XP002280779 ISSN: 0085-2538
- KUNO ET AL: "Molecular cloning of a gene encoding a new type of metalloproteinase-disintegrin family protein with thrombospondin motifs as an inflammation associated gene" JOURNAL OF BIOLOGICAL CHEMISTRY, AMERICAN SOCIETY OF BIOLOGICAL CHEMISTS, BALTIMORE, MD, US, vol. 1, no. 272, 3 January 1997 (1997-01-03), pages 556-562, XP002076038 ISSN: 0021-9258
- TORTORELLA M. ET AL.: 'The thrombospondin motif of aggrecanase-1 (ADAMTS-4) is critical for aggrecan substrate recognition and cleavage' THE JOURNAL OF BIOLOGICAL CHEMISTRY vol. 275, no. 33, August 2000, pages 25791 - 25797, XP002950307
- COLIGE A. ET AL.: 'Human ehlers-danlos syndrome type VII C and bovine dermatosparaxis are caused by mutations in the procollagen I N-proteinase gene' AM. J. HUM. GENET. vol. 65, 1999, pages 308 - 317, XP002950308
- ABBASZADE I. ET AL.: 'Cloning and characterization of ADAMTS11, an aggrecanase from the ADAMTS family' THE JOURNAL OF BIOLOGICAL CHEMISTRY vol. 274, no. 33, August 1999, pages 23443 - 23450, XP002937897
- CARNINCI P. ET AL.: 'Normalization and substraction of cap-trapper-selected cDNAs to prepare full-length cDNA libraries for rapid discovery of new genes' GENOME RESEARCH vol. 10, 2000, pages 1617 - 1630, XP002944079

## Description

### TECHNICAL FIELD

The present invention relates to a novel protease.

### BACKGROUND ART

ADAMTS (A Disintegrin and Metalloprotease with Thrombospondin motif) is a group of molecules containing a disintegrin-like domain, a metallopretease-like domain, and a thrombospondin type I repeated sequence (hereinafter referred to as a TSP-1 repeated sequence). Until now nine human ADAMTS molecules have been reported.

Among the human ADAMTS molecules, it has been shown that ADAMTS4 (aggrecanase-1) and ADAMTS11 (aggrecanase-2) exhibit an activity of selective digestion between the 373rd glutamic acid residue and the 374th alanine residue (between Glu³⁷³-Ala³⁷⁴) of an extracellular substrate aggrecan is, and further, a possibility that they are essential enzymes degrading the extracellular substrate aggrecan in cartilage of arthritis or osteoarthritis was suggested (Tortorella M. D. et al., Science, 284, 1664-1666, 1999; and Abbaszade I. et al., J. Biol. Chem., 274, 23443-23450, 1999). Further, it has been shown that ADAMTS2 (procollagen I N-proteinase) is involved in the conversion from type I procollagen to a mature type thereof as an enzyme cleaving and removing the N-terminal portion of type I procollagen, and plays an important role in the formation of collagen fibers, and that an aberration in the gene thereof is related to VIIC type Ehlers-Danlos syndrome (Colige A. et al., Am. J. Hum. Genet., 65, 308-317, 1999).

Namely, it has been shown that ADAMTS molecules are involved in metabolism such as degradation and maturation of an extracellular matrix (for example, aggrecan, collagen, or the like).

Chronic renal failure is a disease characterized by glomerulosclerosis and mesangial fibrosis. It is considered that a qualitative change and/or a quantitative increase of extracellular matrix components are the main mechanisms of a development and progression thereof. In an experiment using a renal failure model, it was shown that a gene introduction of decorin [a protein which specifically suppresses the activity of transforming growth factor β (TGF-β)] (Isaka Y. et al., Nature Med., 2, 418-423, 1996) and an administration of anti-TGF-β (Ziyadeh F. N. et al., Proc. Natl. Acad. Sci. U.S.A., 97, 8015-8020, 2000; Sharma K. et al., Diabetes, 45, 522-530, 1996; and Border W. A. et al., Nature, 346, 371-374, 1990) were effective. It is considered from these results that a suppression or inhibition of physiological actions of TGF-β leads to a treatment of chronic renal failure.

However, under the current circumstance in which an effective agent for treating chronic renal failure is not known, an agent for inhibiting TGF-β is desired but has not been made readily available until now.

### DISCLOSURE OF INVENTION

TGF-β is a differentiation and growth factor exhibiting various physiological actions. Therefore, it is dangerous to inhibit all physiological actions of TGF-β in a treatment of chronic renal failure in which a long-term administration is foreseen, in view of side effects. It is preferable to suppress or inhibit only a portion involved with a qualitative change and a quantitative increase of extracellular matrix components, among physiological actions of TGF-β.

The object of the present invention is to provide a novel protease which is induced by TGF-β, is involved in metabolism of extracellular matrix, and is useful as a screening tool for an agent for treating chronic renal failure, and a novel polynucleotide encoding the protease.

With the aim of solving the aforementioned problems, the present inventors have conducted intensive studies and, as a result, found a polynucleotide encoding a novel protease consisting of an amino acid sequence consisting of the 1st to 1224th amino acids in the amino acid sequence of SEQ ID NO: 2 and consisting of 1224 amino acid residues from human fetal kidney cDNA. Further, the present inventors found that a partial fragment consisting of 750 amino acid residues at the N-terminus side of the novel protease has an effective protease activity. Furthermore, it was found that (1) the protease is classified into ADAMTS proteases, and thus is considered to be a protease involved in metabolism of extracellular matrix, (2) the protease is actually expressed in the human kidney, (3) an expression thereof is induced by TGF-β in a primary cultured cell from kidney, and (4) an amount of gene thereof expressed increases in a renal failure model animal. From these findings, the present inventors revealed that the protease of the present invention is a polypeptide causative of renal failure, and that, by screening using the polypeptide of the present invention, a substance inhibiting the protease activity thereof, a substance which suppresses or inhibits only a portion involved with a qualitative change and a quantitative increase of extracellular matrix components among physiological actions of TGF-β and is useful as an agent for treating chronic renal failure, can be screened, and completed the present invention.

Accordingly, the present invention relates to:
[1] a polypeptide exhibiting a protease activity and comprising (1) an amino acid sequence consisting of a 1st to 750th amino acids in an amino acid sequence of SEQ ID NO: 2, (2) an amino acid sequence in which 1 to 10 amino acids are deleted, substituted, and/or inserted in an amino acid sequence consisting of a 1st to 750th amino acids in an amino acid sequence of SEQ ID NO: 2, or (3) an amino acid sequence in which 1 to 10 amino acids are deleted, substituted, and/or inserted in an amino acid sequence consisting of a 1st to 1224th amino acids in an amino acid sequence of SEQ ID NO: 2;
[2] a polypeptide comprising an amino acid sequence consisting of a 1st to 750th amino acids in an amino acid sequence of SEQ ID NO: 2, and exhibiting a protease activity;
[3] a polypeptide exhibiting a protease activity and consisting of (1) an amino acid sequence in which 1 to 10 amino acids are deleted, substituted, and/or inserted in an amino acid sequence consisting of a 1st to 750th amino acids in an amino acid sequence of SEQ ID NO: 2, or (2) an amino acid sequence in which 1 to 10 amino acids are deleted, substituted, and/or inserted in an amino acid sequence consisting of a 1st to 1224th amino acids in an amino acid sequence of SEQ ID NO: 2;
[6] a polypeptide consisting of (1) a 1st to 750th amino acids in an amino acid sequence of SEQ ID NO: 2, or (2) a 1st to 1224th amino acids in an amino acid sequence of SEQ ID NO: 2;
[7] a polynucleotide encoding the polypeptide of the items [1] to [6];
[8] an expression vector comprising the polynucleotide of the item [7];
[9] a cell transfected with the expression vector of the item [8];
[10] an antibody or a fragment thereof, which binds to the polypeptide of the items [1] to [6];
[11] a process for producing the polypeptide of the items [1] to [6], comprising the steps of:
   culturing the cell of the item [9], and
   recovering the polypeptide of the items [1] to [6];
[12] a method for detecting whether or not a compound to be tested inhibits a protease activity of the polypeptide of the items [1] to [6], comprising the steps of:
   bringing into contact (1) the polypeptide, (2) α₂-macroglobulin, and (3) the compound to be tested, and
   analyzing whether or not the polypeptide and α₂-macroglobulin form a complex which is not dissociated by SDS and/or a reducing agent;
[13] a method for screening a substance which inhibits a protease activity of the polypeptide of the items [1] to [6], comprising the steps of:
   detecting by the method of the item [12], and
   selecting a substance inhibiting the protease activity;
[14] a method for screening a substance for treating chronic renal failure by the method of the item [13]; and

The term "protease activity" as used herein means a property in which a complex which is not dissociated by sodium dodecyl sulfate (SDS) and/or a reducing agent can be formed with α₂-macroglobulin, a protease-inhibiting protein present in a serum.

### BEST MODE FOR CARRYING OUT THE INVENTION

The present invention will be explained in detail hereinafter.

### [1] The polypeptide of the present invention

The polypeptide of the present invention includes
(1) a polypeptide comprising an amino acid sequence consisting of the 1st to 750th amino acids in the amino acid sequence of SEQ ID NO: 2;
(2) a polypeptide comprising an amino acid sequence in which 1 to 10 amino acids in total are deleted, substituted, and/or inserted at one or plural positions in the amino acid sequence consisting of the 1st to 750th amino acids in the amino acid sequence of SEQ ID NO: 2, and exhibiting the protease activity (hereinafter referred to as a variation functionally equivalent); and
(3) a polypeptide comprising the amino acid sequence consisting of the 1st to 750th amino acids in the amino acid sequence of SEQ ID NO: 2 or the amino acid sequence consisting of the 1st to 1224th amino acids in the amino acid sequence of SEQ ID NO: 2, and exhibiting the protease activity (hereinafter referred to as a homologous polypeptide).

The "polypeptide comprising the amino acid sequence consisting of the 1st to 750th amino acids in the amino acid sequence of SEQ ID NO: 2" as the polypeptide of the present invention is not limited, so long as it is a polypeptide comprising the amino acid sequence consisting of the 1st to 750th amino acids in the amino acid sequence of SEQ ID NO: 2, and exhibiting the protease activity. It includes, for example,
(1a) a polypeptide consisting of the 1st to 750th amino acids in an amino acid sequence of SEQ ID NO: 2;
(1b) a fusion polypeptide having an amino acid sequence in which an appropriate marker sequence or the like is added to the N-terminus and/or the C-terminus of the amino acid sequence consisting of the 1st to 750th amino acids in the amino acid sequence of SEQ ID NO: 2, and exhibiting the protease activity;
(1c) a polypeptide having an amino acid sequence in which an amino acid sequence consisting of the 751st to 1224th amino acids in the amino acid sequence of SEQ ID NO: 2, or an amino acid sequence in which 1 to 473 amino acids are deleted from the C-terminus thereof, is added to the C-terminus of the amino acid sequence consisting of the 1st to 750th amino acids in the amino acid sequence of SEQ ID NO: 2 (i.e., the amino acid of the C-terminus is any one of the 751st to 1224th amino acids; hereinafter referred to as "the amino acid sequence of SEQ ID NO: 2 or a C-terminus-deleted sequence thereof");
(1d) a fusion polypeptide having an amino acid sequence in which an appropriate marker sequence or the like is added to the N-terminus and/or the C-terminus of the amino acid sequence of SEQ ID NO: 2 or the C-terminus-deleted sequence thereof, and exhibiting the protease activity; and the like.

A method for confirming whether or not a polypeptide to be tested (hereinafter referred to as a test polypeptide) "exhibits the protease activity" as used herein (hereinafter sometimes referred to as a "method for confirming the protease activity") is not particularly limited, so long as it can be confirmed whether or not the test polypeptide exhibits "a property in which a complex which is not dissociated by SDS and/or a reducing agent can be formed with α₂-macroglobulin, a protease-inhibiting protein present in a serum". It can be confirmed, for example, by bringing the test polypeptide into contact with α₂-macroglobulin, a protease-inhibiting protein present in a serum, and then analyzing whether or not a complex which is not dissociated by SDS and/or a reducing agent [such as 2-mercaptoethanol (2-ME)] is formed, more particularly by a method described in Example 4.

It is known that α₂-macroglobulin is a protease-inhibiting protein present in a serum and can form a complex with various proteases. It is known that the formation of the complex depends on a protease activity, and that the formed complex is formed by an amide bond of protease and α₂-macroglobulin, and thus is not dissociated by SDS or a reducing agent such as 2-ME (Feinman R. D. et al., Ann. New York Acad. Sci., 737, 245-266, 1994; and Kuno K. et al., J. Biol. Chem., 274, 18821-18826, 1999).

The above polypeptide (1a), i.e., "the polypeptide consisting of the 1st to 750th amino acids in the amino acid sequence of SEQ ID NO: 2" is a novel protease consisting of 750 amino acid residues and exhibiting the protease activity. The polypeptide (1a) corresponds to a partial polypeptide of "the polypeptide consisting of the 1st to 1224th amino acids in the amino acid sequence of SEQ ID NO: 2".

As the marker sequence in the polypeptide of the present invention, for example, a sequence for easily carrying out confirmation of polypeptide expression, confirmation of intracellular localization thereof, purification thereof, or the like may be used. As the sequence, there may be mentioned, for example, the FLAG tag, the hexa-histidine tag, the hemagglutinin tag, the myc epitope, or the like.

The variation functionally equivalent of the present invention is not particularly limited, so long as it is a polypeptide comprising an amino acid sequence in which 1 to 10, preferably 1 to 7, more preferably 1 to 5 (for example, one to several amino acids) are deleted, substituted, and/or inserted at one or plural positions in the amino acid sequence consisting of the 1st to 750th amino acids in the amino acid sequence of SEQ ID NO: 2, and exhibiting the protease activity. Further, an origin of the variation functionally equivalent is not limited to a human.

The variation functionally equivalent of the present invention includes, for example, human variations of the polypeptide consisting of the 1st to 750th amino acids in the amino acid sequence of SEQ ID NO: 2 and variations functionally equivalent derived from organisms other than human (such as mouse, rat, hamster, or dog), and further polypeptides prepared using polynucleotides obtained by artificially modifying polynucleotides encoding these native polypeptides (i.e., human variations or variations functionally equivalent derived from organisms other than human) or polynucleotides encoding the polypeptide consisting of the 1st to 750th amino acids in the amino acid sequence of SEQ ID NO: 2 by genetic engineering techniques. The term "variation" as used herein means individual differences between the same polypeptides in the same species or differences between homologous polypeptides in several species.

Human variations of the polypeptide consisting of the 1st to 750th amino acids in the amino acid sequence of SEQ ID NO: 2 or variations functionally equivalent derived from organisms other than a human may be obtained by those skilled in the art in accordance with the information of a base sequence (for example, the base sequence of SEQ ID NO: 1) of a polynucleotide encoding the polypeptide consisting of the 1st to 750th amino acids in the amino acid sequence of SEQ ID NO: 2. In this connection, genetic engineering techniques may be generally performed in accordance with known methods (for example, Sambrook, J. et al., "Molecular Cloning-A Laboratory Manual", Cold Spring Harbor Laboratory, NY, 1989).

For example, an appropriate probe or appropriate primers are designed in accordance with the information of a base sequence of a polynucleotide encoding the polypeptide consisting of the 1st to 750th amino acids in the amino acid sequence of SEQ ID NO: 2. A polymerase chain reaction (PCR) method (Saiki, R. K. et al., Science, 239, 487-491, 1988) or a hybridization method is carried out using a sample (for example, total RNA or an mRNA fraction, a cDNA library, or a phage library) prepared from an organism (for example, a mammal such as human, mouse, rat, hamster, or dog) of interest and the primers or the probe to obtain a polynucleotide encoding the polypeptide. A desired polypeptide may be obtained by expressing the resulting polynucleotide in an appropriate expression system and confirming that the expressed polypeptide exhibits the protease activity by, for example, the method described in Example 4.

Further, the polypeptide artificially modified by genetic engineering techniques may be obtained by, for example, the following procedure. A gene encoding the polypeptide may be obtained by a conventional method, for example, site-directed mutagenesis (Mark, D. F. et al., Proc. Natl. Acad. Sci. USA, 81, 5662-5666, 1984). A desired polypeptide may be obtained by expressing the resulting polynucleotide in an appropriate expression system and confirming that the expressed polypeptide exhibits the protease activity by, for example, the method described in Example 4.

The variation functionally equivalent of the present invention includes, for example,
(2a) a polypeptide having an amino acid sequence in which 1 to 10 amino acids in total are deleted, substituted, and/or inserted at one or plural positions in the amino acid sequence consisting of the 1st to 750th amino acids in the amino acid sequence of SEQ ID NO: 2, and exhibiting the protease activity;
(2b) a fusion polypeptide having an amino acid sequence in which 1 to 10 amino acids in total are deleted, substituted, and/or inserted at one or plural positions in the amino acid sequence consisting of the 1st to 750th amino acids in the amino acid sequence of SEQ ID NO: 2 and an appropriate marker sequence or the like is added to the N-terminus and/or the C-terminus thereof, and exhibiting the protease activity;
(2c) a polypeptide having an amino acid sequence in which 1 to 10 amino acids in total are deleted, substituted, and/or inserted at one or plural positions in the amino acid sequence consisting of the 1st to 750th amino acids in the amino acid sequence of SEQ ID NO: 2, and to the C-terminus thereof, an amino acid sequence consisting of the 751st to 1224th amino acids in the amino acid sequence of SEQ ID NO: 2, or an amino acid sequence in which 1 to 473 amino acids are deleted from the C-terminus thereof, is added, and exhibiting the protease activity; and
(2d) a polypeptide having an amino acid sequence in which 1 to 10 amino acids in total are deleted, substituted, and/or inserted at one or plural positions in the amino acid sequence consisting of the 1st to 750th amino acids in the amino acid sequence of SEQ ID NO: 2, and to the C-terminus thereof, an amino acid sequence consisting of the 751st to 1224th amino acids in the amino acid sequence of SEQ ID NO: 2, or an amino acid sequence in which 1 to 473 amino acids are deleted from the C-terminus thereof, is added, and an appropriate marker sequence or the like is further added to the N-terminus and/or the C-terminus thereof, and exhibiting the protease activity.

As above, the polypeptide of the present invention is explained, but as the polypeptide of the present invention, "the polypeptide consisting of the 1st to 750th amino acids in the amino acid sequence of SEQ ID NO: 2",
"the polypeptide consisting of the 1st to 1224th amino acids in the amino acid sequence of SEQ ID NO: 2",
"a polypeptide consisting of an amino acid sequence in which 1 to 10 (preferably 1 to 7, more preferably 1 to 5) in total are deleted, substituted, inserted, and/or added at one or plural positions in the amino acid sequence consisting of the 1st to 750th amino acids in the amino acid sequence of SEQ ID NO: 2, and exhibiting the protease activity", or
"a polypeptide consisting of an amino acid sequence in which 1 to 10 (preferably 1 to 7, more preferably 1 to 5) in total are deleted, substituted, inserted, and/or added at one or plural positions in the amino acid sequence consisting of the 1st to 750th amino acids in the amino acid sequence of SEQ ID NO: 2, and exhibiting the protease activity" is preferable, and
"the polypeptide consisting of the 1st to 750th amino acids in the amino acid sequence of SEQ ID NO: 2", or
"the polypeptide consisting of the 1st to 1224th amino acids in the amino acid sequence of SEQ ID NO: 2" is more preferable.

### [2] The polynucleotide of the present invention

The polynucleotide of the present invention is not particularly limited, so long as it encodes the polypeptide of the present invention. As the polynucleotide of the present invention, there may be mentioned, for example, a polynucleotide comprising a base sequence consisting of the 1st to 2250th bases in the base sequence of SEQ ID NO: 1. A polynucleotide consisting of the 1st to 2250th bases in the base sequence of SEQ ID NO: 1 is most preferable. In this connection, the term "polynucleotide" as used herein includes both DNA and RNA.

A method for producing the polynucleotide of the present invention is not particularly limited, but there may be mentioned, for example, (1) a method using PCR, (2) a method using conventional genetic engineering techniques (i.e., a method for selecting a transformant comprising a desired cDNA from strains transformed with a cDNA library), or (3) a chemical synthesis method. These methods will be explained in this order hereinafter.

In the method using PCR of the item (1), the polynucleotide of the present invention may be produced, for example, by the following procedure.

mRNA is extracted from human cells or tissue capable of producing the polypeptide of the present invention. A pair of primers, between which full-length mRNA corresponding to the polypeptide of the present invention or a partial region of the mRNA is located, is synthesized on the basis of the base sequence of a polynucleotide encoding the polynucleotide of the present invention. Full-length cDNA encoding the polypeptide of the present invention or a part of the cDNA may be obtained by performing a reverse transcriptase-polymerase chain reaction (RT-PCR) using the extracted mRNA as a template.

More particularly, total RNA containing mRNA encoding the polypeptide of the present invention is extracted by a known method from cells or tissue capable of producing the polypeptide of the present invention. As an extraction method, there may be mentioned, for example, a guanidine thiocyanate-hot phenol method, a guanidine thiocyanate-guanidine hydrochloride method, or a guanidine thiocyanate-cesium chloride method. The guanidine thiocyanate-cesium chloride method is preferably used. The cells or tissue capable of producing the polypeptide of the present invention may be identified, for example, by a northern blotting method using a polynucleotide or a part thereof encoding the polypeptide of the present invention or a western blotting method using an antibody specific for the polypeptide of the present invention.

Next, the extracted mRNA is purified. Purification of the mRNA may be made in accordance with a conventional method. For example, the mRNA may be purified by adsorption and elution using an oligo(dT)-cellulose column. The mRNA may be further fractionated by, for example, a sucrose density gradient centrifugation, if necessary. Alternatively, commercially available extracted and purified mRNA may be used without carrying out the extraction of the mRNA.

Next, the first-strand cDNA is synthesized by carrying out a reverse transcriptase reaction of the purified mRNA in the presence of a random primer, an oligo dT primer, and/or a custom primer. This synthesis may be carried out in accordance with a conventional method. The resulting first-strand cDNA is subjected to PCR using two primers between which a full-length or a partial region of the polynucleotide of interest is located, thereby amplifying the cDNA of interest. The resulting DNA is fractionated by, for example, an agarose gel electrophoresis. The DNA fragment of interest may be obtained by carrying out a digestion of the DNA with restriction enzymes and subsequent ligation, if necessary.

In the method using conventional genetic engineering techniques of the item (2), the polynucleotide of the present invention may be produced, for example, by the following procedure.

First, single-stranded cDNA is synthesized by using reverse transcriptase from mRNA prepared by the above-mentioned PCR method as a template, and then double-stranded cDNA is synthesized from the single-stranded cDNA. As this method, there may be mentioned, for example, an S1 nuclease method (Efstratiadis, A. et al., Cell, 7, 279-288, 1976), a Land method (Land, H. et al., Nucleic Acids Res., 9, 2251-2266, 1981), an O. Joon Yoo method (Yoo, O. J. et al., Proc. Natl. Acad. Sci. USA, 79, 1049-1053, 1983), and an Okayama-Berg method (Okayama, H. and Berg, P., Mol. Cell. Biol., 2, 161-170, 1982).

Next, a recombinant plasmid comprising the double-stranded cDNA is prepared and introduced into an Escherichia coli strain, such as DH 5α, HB101, or JM109, thereby transforming the strain. A transformant is selected using a drug resistance against, for example, tetracycline, ampicillin, or kanamycin as a marker. When the host cell is E. coli, transformation of the host cell may be carried out, for example, by the method of Hanahan (Hanahan, D. J., Mol. Biol., 166, 557-580, 1983); namely, a method in which the recombinant DNA is added to competent cells prepared in the presence of CaCl₂, MgCl₂, or RbCl. Further, as a vector other than a plasmid, a phage vector such as a lambda system may be used.

As a method for selecting a transformant containing the cDNA of interest from the resulting transformants, various methods such as (i) a method for screening a transformant using a synthetic oligonucleotide probe, (ii) a method for screening a transformant using a probe produced by PCR, (iii) a method for screening a transformant using an antibody against the polypeptide of the present invention, or (iv) a method for screening a transformant using a selective hybridization translation system, may be used.

In the method of the item (i) for screening a transformant using a synthetic oligonucleotide probe, the transformant containing the cDNA of interest may be selected, for example, by the following procedure.

An oligonucleotide which corresponds to the whole or a part of the polypeptide of the present invention is synthesized (in this case, it may be either a nucleotide sequence taking the codon usage into consideration or a plurality of nucleotide sequences as a combination of possible nucleotide sequences, and in the latter case, their numbers can be reduced by including inosine) and, using this oligonucleotide as a probe (labeled with ³²P or ³³P), hybridized with a nitrocellulose filter or a polyamide filter on which DNAs of the transformants are denatured and fixed, to screen and select resulting positive strains.

In the method of the item (ii) for screening a transformant using a probe produced by PCR, the transformant containing the cDNA of interest may be selected, for example, by the following procedure.

Oligonucleotides of a sense primer and an antisense primer corresponding to a part of the polypeptide of the present invention are synthesized, and a DNA fragment encoding the whole or a part of the polypeptide of interest is amplified by carrying out PCR using these primers in combination. As a template DNA used in this method, cDNA synthesized by a reverse transcription reaction from mRNA of cells capable of producing the polypeptide of the present invention, or genomic DNA, may be used. The resulting DNA fragment is labeled with ³²P or ³³P, and a transformant containing the cDNA of interest is selected by carrying out a colony hybridization or a plaque hybridization using this fragment as a probe.

In the method of the item (iii) for screening a transformant using an antibody against the polypeptide of the present invention, the transformant containing the cDNA of interest may be selected, for example, by the following procedure.

First, cDNA is integrated into an expression vector, and polypeptides are produced into a culture supernatant, inside the cells, or on the cell surface of transformants. A transformant containing the cDNA of interest is selected by detecting a strain producing the desired polypeptide using an antibody against the polypeptide of the present invention and a second antibody against the first antibody.

In the method of the item (iv) for screening a transformant using a selective hybridization translation system, the transformant containing the cDNA of interest may be selected, for example, by the following procedure.

First, cDNA obtained from each transformant is blotted on, for example, a nitrocellulose filter and hybridized with mRNA prepared from cells capable of producing the polypeptide of the present invention, and then the mRNA bound to the cDNA is dissociated and recovered. The recovered mRNA is translated into a polypeptide-in an appropriate polypeptide translation system, for example, injection into Xenopus oocytes or a cell-free system such as a rabbit reticulocyte lysate or a wheat germ. A transformant containing the cDNA of interest is selected by detecting it with the use of an antibody against the polypeptide of the present invention.

A method for collecting the polynucleotide of the present invention from the resulting transformant of interest can be carried out in accordance with a known method (for example, Sambrook, J. et al., "Molecular Cloning-A Laboratory Manual", Cold Spring Harbor Laboratory, NY, 1989). For example, it may be carried out by separating a fraction corresponding to the plasmid DNA from cells and cutting out the cDNA region from the plasmid DNA.

In the chemical synthesis method of the item (3), the polynucleotide of the present invention may be produced, for example, by binding DNA fragments produced by a chemical synthesis method. Each DNA can be synthesized using a DNA synthesizer [for example, Oligo 1000M DNA Synthesizer (Beckman) or 394 DNA/RNA Synthesizer (Applied Biosystems)].

Further, the polynucleotide of the present invention may be produced by nucleic acid chemical synthesis in accordance with a conventional method such as a phosphite triester method (Hunkapiller, M. et al., Nature, 10, 105-111, 1984), based on the information on the polypeptide of the present invention. In this connection, codons for each amino acid are known and can be optionally selected and determined by the conventional method, for example, by taking a codon usage of each host to be used into consideration (Crantham, R. et al., Nucleic Acids Res., 9, r43-r74, 1981). Further, a partial modification of codons of these base sequences can be carried out in accordance with a conventional method, such as site directed mutagenesis which uses a primer comprised of a synthetic oligonucleotide coding for a desired modification (Mark, D. F. et al., Proc. Natl. Acad. Sci. USA, 81, 5662-5666, 1984).

Determination of the DNA sequences obtained by the above-mentioned methods can be carried out by, for example, a Maxam-Gilbert chemical modification method (Maxam, A. M. and Gilbert, W., "Methods in Enzymology", 65, 499-559, 1980) or a dideoxynucleotide chain termination method (Messing, J. and Vieira, J., Gene, 19, 269-276, 1982).

### [3] The expression vector and the cell of the present invention

An isolated polynucleotide of the present invention is re-integrated into an appropriate vector DNA and a eucaryotic or procaryotic host cell may be transfected by the resulting expression vector. Further, it is possible to express the polynucleotide in a desired host cell, by introducing an appropriate promoter and a sequence related to the gene expression into the vector.

The expression vector of the present invention is not particularly limited, so long as it comprises the polynucleotide of the present invention. As the expression vector, there may be mentioned, for example, an expression vector obtained by introducing the polynucleotide of the present invention into a known expression vector appropriately selected in accordance with a host cell to be used.

The cell of the present invention is not particularly limited, so long as it is transfected with the expression vector of the present invention and comprises the polynucleotide of the present invention. The cell of the present invention may be, for example, a cell in which the polynucleotide is integrated into a chromosome of a host cell, or a cell containing the polynucleotide as an expression vector comprising polynucleotide. Further, the cell of the present invention may be a cell expressing the polypeptide of the present invention, or a cell not expressing the polypeptide of the present invention. The cell of the present invention may be obtained by, for example, transfecting a desired host cell with the expression vector of the present invention.

In the eucaryotic host cells, for example, cells of vertebrates, insects, and yeast are included. As the vertebral cell, there may be mentioned, for example, a simian COS cell (Gluzman, Y., Cell, 23, 175-182, 1981), a dihydrofolate reductase defective strain of a Chinese hamster ovary cell (CHO) (Urlaub, G. and Chasin, L. A., Proc. Natl. Acad. Sci. USA, 77, 4216-4220, 1980), a human fetal kidney derived HEK293 cell, or a 293-EBNA cell (Invitrogen) obtained by introducing an EBNA-1 gene of Epstein Barr Virus into HEK293 cell.

As an expression vector for a vertebral cell, a vector containing a promoter positioned upstream of the gene to be expressed, an RNA splicing site, a polyadenylation site, a transcription termination sequence, and the like may be generally used. The vector may further contain a replication origin, if necessary. As the expression vector, there may be mentioned, for example, pSV2dhfr containing an SV40 early promoter (Subramani, S. et al., Mol. Cell. Biol., 1, 854-864, 1981), pEF-BOS containing a human elongation factor promoter (Mizushima, S. and Nagata, S., Nucleic Acids Res., 18,5322, 1990), or pCEP4 containing a cytomegalovirus promoter (Invitrogen).

When the 293-EBNA cell is used as the host cell, for example, pCEP4 (Invitrogen) containing a replication origin of Epstein Barr Virus and capable of performing an autonomous replication in the 293-EBNA cell may be used as the expression vector.

When the COS cell is used as the host cell, a vector which has an SV40 replication origin, can perform an autonomous replication in the COS cell, and has a transcription promoter, a transcription termination signal, and an RNA splicing site, may be used as the expression vector. As the vector, there may be mentioned, for example, pME18S (Maruyama, K. and Takebe, Y., Med. Immunol., 20, 27-32, 1990), pEF-BOS (Mizushima, S. and Nagata, S., Nucleic Acids Res., 18, 5322, 1990), or pCDM8 (Seed, B., Nature, 329, 840-842, 1987).

The expression vector may be incorporated into COS cells by, for example, a DEAE-dextran method (Luthman, H. and Magnusson, G., Nucleic Acids Res., 11, 1295-1308, 1983), a calcium phosphate-DNA co-precipitation method (Graham, F. L. and van der Ed, A. J., Virology, 52, 456-457, 1973), a method using a commercially available transfection reagent (for example, FuGENE^{™}6 Transfection Reagent; Boeringer Mannheim), or an electroporation method (Neumann, E. et al., EMBO J., 1, 841-845, 1982).

When the CHO cell is used as the host cell, a transfected cell capable of stably producing the polypeptide of the present invention can be obtained by carrying out co-transfection of an expression vector comprising the polynucleotide encoding the polypeptide of the present invention, together with a vector capable of expressing a neo gene which functions as a G418 resistance marker, such as pRSVneo (Sambrook, J. et al., "Molecular Cloning-A Laboratory Manual", Cold Spring Harbor Laboratory, NY, 1989) or pSV2-neo (Southern, P. J. and Berg, P., J. Mol. Appl. Genet., 1, 327-341,1982), and selecting a G418 resistant colony.

The cell of the present invention may be cultured in accordance with the conventional method [for example, "Shin Seikagaku Jikken Koza 18, Saibou Baiyou Gijyutsu (Japanese Biochemical Society)", Tokyo Kagaku Dojin, 1990], and the polypeptide of the present invention is produced outside the cells. As a medium to be used in the culturing, a medium commonly used in a desired host cell may be appropriately selected. In the case of the COS cell, for example, a medium such as an RPMI-1640 medium or a Dulbecco's modified Eagle's minimum essential medium (DMEM) may be used, by supplementing it with a serum component such as fetal bovine serum (FBS) if necessary. In the case of the 293-EBNA cell, a medium such as a Dulbecco's modified Eagle's minimum essential medium (DMEM) with a serum component such as fetal bovine serum (FBS) and G418 may be used.

The polypeptide of the present invention produced outside the cell of the present invention by culturing the cells may be separated and purified therefrom by various known separation techniques [for example, Okada, M. and Miyazaki K., "Kaitei, Tanpakushitsu Jikken Noto, Jyo-Ge (Revision, Notebook for Protein Experiments)", Yodo-sha 1999] making use of the physical properties, chemical properties and the like of the polypeptide. More particularly, the polypeptide of the present invention may be purified by treating a culture liquid containing the polypeptide of the present invention with a commonly used treatment, for example, a treatment with a protein precipitant, ultrafiltration, various liquid chromatography techniques such as molecular sieve chromatography (gel filtration), adsorption chromatography, ion exchange chromatography, affinity chromatography, or high performance liquid chromatography (HPLC), or dialysis, or a combination thereof.

When the polypeptide of the present invention is expressed as a fusion protein with a marker sequence in frame, identification of the expression of the polypeptide of the present invention, purification thereof, or the like may be easily carried out. As the marker sequence, there may be mentioned, for example, a FLAG tag, a hexa-histidine tag, a hemagglutinin tag, or a myc epitope. Further, by inserting a specific amino acid sequence recognized by a protease such as enterokinase, factor Xa, or thrombin between the marker sequence and the polypeptide of the present invention, the marker sequence may be removed by the protease.

### [4] The detection method and the screening method of the present invention

It is possible to detect whether or not a compound to be tested inhibits the protease activity of the polypeptide of the present invention, using the polypeptide of the present invention. Further, using this detection method of the present invention, it is possible to screen a substance inhibiting the protease activity of the polypeptide of the present invention. MDTS9 (Metalloprotease and Disintegrin with Thrombospondin type-1 repeats 9), the polypeptide of the present invention, is considered to be an ADAMTS protease from its sequence, and thus to be a protease which is involved in metabolism of extracellular matrix. MDTS9 is a protein expressed in kidney as shown in Examples 5 and 8, and an ADAMTS protease induced by TGF-β as shown in Example 6. Therefore, a substance inhibiting the protease activity of the polypeptide of the present invention is useful as an agent for treating chronic renal failure in which a long-term administration is foreseen, because it is highly possible that the substance suppresses or inhibits only a portion which is involved in a qualitative change and a quantitative increase of extracellular matrix components, among physiological actions of TGF-β. Further, the polypeptide of the present invention per se may be used as a tool for screening a substance inhibiting the protease activity of the polypeptide of the present invention or a substance for treating chronic renal failure.

Compounds to be tested which may be applied to the detection method or screening method of the present invention are not particularly limited, but there may be mentioned, for example, various known compounds (including peptides) registered in chemical files, compounds obtained by combinatorial chemistry techniques (Terrett, N. K. et al., Tetrahedron, 51, 8135-8137, 1995) or conventional synthesis techniques, or random peptides prepared by employing a phage display method (Felici, F. et al., J. Mol. Biol., 222, 301-310, 1991) or the like. These known compounds include compounds (including peptides) known to exhibit an activity inhibiting protease but not known to inhibit the protease activity of the polypeptide of the present invention. In addition, culture supernatants of microorganisms, natural components derived from plants or marine organisms, or animal tissue extracts may be used as the test compounds for screening. Further, compounds (including peptides) obtained by chemically or biologically modifying compounds (including peptides) selected by the screening method of the present invention may be used.

The detection method of the present invention comprises the steps of:
bringing into contact (1) the polypeptide of the present invention, (2) α₂-macroglobulin, and (3) a compound to be tested, and
analyzing whether or not the polypeptide of the present invention and α₂-macroglobulin form a complex which is not dissociated by SDS and/or a reducing agent (such as 2-ME).

The detection method of the present invention may be carried out by a method similar to the above-mentioned method for confirming the protease activity, except that the polypeptide of the present invention, α₂-macroglobulin, and the test compound are brought into contact with each other instead of bringing the test polypeptide into contact with α₂-macroglobulin. Namely, in the detection method of the present invention, it is detected whether or not the test compound inhibits the protease activity of the polypeptide of the present invention by bringing into contact the polypeptide of the present invention, α₂-macroglobulin, and the test polypeptide, and then analyzing whether or not the polypeptide of the present invention and α₂-macroglobulin form a complex which is not dissociated by SDS and/or a reducing agent (such as 2-ME) in the presence of the test compound. When the polypeptide of the present invention and α₂-macroglobulin do not form a complex which is not dissociated by SDS and/or a reducing agent (such as 2-ME) in the presence of the test compound, or the degree of the formation is decreased, it is possible to confirm that the test compound inhibits the protease activity of the polypeptide of the present invention.

In the screening method of the present invention, a substance inhibiting the protease activity of the polypeptide of the present invention or a substance for treating chronic renal failure is selected, on the basis of the results obtained by detecting whether or not the test compound inhibits the protease activity of the polypeptide of the present invention using the detecting method of the present invention. More particularly, for example, when the polypeptide of the present invention, α₂-macroglobulin, and the test compound are brought into contact with each other, a substance inhibiting the protease activity of the polypeptide of the present invention or a substance for treating chronic renal failure can be selected on the basis of the presence or degree of formation of the complex of the polypeptide of the present invention and α₂-macroglobulin in the presence of the test compound. When the polypeptide of the present invention and α₂-macroglobulin do not form a complex which is not dissociated by SDS and/or a reducing agent (such as 2-ME) in the presence of the test compound, or the degree of the formation is decreased, it is possible to confirm that the test compound is a substance inhibiting the protease activity of the polypeptide of the present invention or a substance for treating chronic renal failure.

### [6] The antibody and the fragment thereof of the present invention

An antibody, such as a polyclonal antibody or a monoclonal antibody, which reacts with the polypeptide of the present invention may be obtained by directly administering the polypeptide of the present invention or a fragment thereof to various animals. Alternatively, it may be obtained by a DNA vaccine method (Raz, E. et al., Proc. Natl. Acad. Sci. USA, 91, 9519-9523, 1994; or Donnelly, J. J. et al., J. Infect. Dis., 173, 314-320, 1996), using a plasmid into which a polynucleotide encoding the polypeptide of the present invention is inserted.

The polyclonal antibody may be produced from a serum or eggs of an animal such as a rabbit, a rat, a goat, or a chicken, in which the animal is immunized and sensitized by the polypeptide of the present invention or a fragment thereof emulsified in an appropriate adjuvant (for example, Freund's complete adjuvant) by intraperitoneal, subcutaneous, or intravenous administration. The polyclonal antibody may be separated and purified from the resulting serum or eggs in accordance with conventional methods for polypeptide isolation and purification. Examples of the separation and purification methods include, for example, centrifugal separation, dialysis, salting-out with ammonium sulfate, or a chromatographic technique using such as DEAE-cellulose, hydroxyapatite, protein A agarose, and the like.

The monoclonal antibody may be easily produced by those skilled in the art, according to, for example, a cell fusion method of Kohler and Milstein (Kohler, G. and Milstein, C., Nature, 256, 495-497, 1975).

A mouse is immunized intraperitoneally, subcutaneously, or intravenously several times at an interval of a few weeks by a repeated inoculation of emulsions in which the polypeptide of the present invention or a fragment thereof is emulsified into a suitable adjuvant such as Freund's complete adjuvant. Spleen cells are removed after the final immunization, and then fused with myeloma cells to prepare hybridomas.

As a myeloma cell for obtaining a hybridoma, a myeloma cell having a marker such as a deficiency in hypoxanthine-guanine phosphoribosyltransferase or thymidine kinase (for example, mouse myeloma cell line P3X63Ag8.U1) may be used. As a fusing agent, polyethylene glycol may be used. As a medium for preparation of hybridomas, for example, a commonly used medium such as an Eagle's minimum essential medium, a Dulbecco's modified minimum essential medium, or an RPMI-1640 medium may be used by adding properly 10 to 30% of a fetal bovine serum. The fused strains may be selected by a HAT selection method. A culture supernatant of the hybridomas is screened by a well-known method such as an ELISA method or an immunohistological method, to select hybridoma clones secreting the antibody of interest. The monoclonality of the selected hybridoma is guaranteed by repeating subcloning by a limiting dilution method. Antibodies in an amount which may be purified are produced by culturing the resulting hybridomas in a medium for 2 to 4 days, or in the peritoneal cavity of a pristane-pretreated BALB/c strain mouse for 10 to 20 days.

The resulting monoclonal antibodies in the culture supernatant or the ascites may be separated and purified by conventional polypeptide isolation and purification methods. Examples of the separation and purification methods include, for example, centrifugal separation, dialysis, salting-out with ammonium sulfate, or chromatographic technique using such as DEAE-cellulose, hydroxyapatite, protein A agarose, and the like.

Further, the monoclonal antibodies or the antibody fragments containing a part thereof may be produced by inserting the whole or a part of a gene encoding the monoclonal antibody into an expression vector and introducing the resulting expression vector into appropriate host cells (such as E. coli, yeast, or animal cells).

Antibody fragments comprising an active part of the antibody such as F(ab')₂, Fab, Fab', or Fv may be obtained by a conventional method, for example, by digesting the separated and purified antibodies (including polyclonal antibodies and monoclonal antibodies) with a protease such as pepsin or papain, and separating and purifying the resulting fragments by standard polypeptide isolation and purification methods.

Further, an antibody which reacts to the polypeptide of the present invention may be obtained in a form of single chain Fv or Fab in accordance with a method of Clackson et al. or a method of Zebedee et al. (Clackson, T. et al., Nature, 352, 624-628, 1991; or Zebedee, S. et al., Proc. Natl. Acad. Sci. USA, 89, 3175-3179, 1992). Furthermore, a humanized antibody may be obtained by immunizing a transgenic mouse in which mouse antibody genes are substituted with human antibody genes (Lonberg, N. et al., Nature, 368, 856-859, 1994).

### EXAMPLES

The present invention now will be further illustrated by, but is by no means limited to, the following Examples. The procedures were performed in accordance with the known methods (Sambrook, J., et al., "Molecular Cloning - A Laboratory Manual", Cold Spring Harbor Laboratory, NY, 1989), unless otherwise specified.

### Example 1: Preparation of expression vector having FLAG added to C-terminus

An expression vector pCEP4d wherein the Estein-Barr virus EBNA1 expression unit has been removed is constructed by digesting plasmid pCEP4 (manufactured by Invitrogen) with restriction enzymes ClaI and NsiI, blunt-ending and then self-ligating. The resulting expression vector pCEP4d was digested with restriction enzymes, NheI and BamHI, and the resulting DNA fragment of approximately 7.7 kbp was extracted from agarose gel, to which the double stranded oligonucleotide prepared by annealing the oligonucleotide consisting of the base sequence of SEQ ID NO: 3 and the oligonucleotide consisting of the base sequence of SEQ ID NO: 4 was then inserted to construct the expression vector pCEP4d-FLAG. The base sequence of the resulting expression vector was analyzed to confirm that the desired sequence was included therein.

A PCR was carried out, using the expression vector pCEP4d-FLAG as a template, the oligonucleotide consisting of the base sequence of SEQ ID NO: 5 and the oligonucleotide consisting of the base sequence of SEQ ID NO: 6 as primers, and PyroBest DNA polymerase (PyroBest^{™}; manufactured by Takara-shuzo). In the PCR, a thermal denaturing reaction was performed first at 94°C for 2 minutes. Then, a cycle reaction composed of treatments at 94°C for 30 seconds, at 55°C for 30 seconds and at 72°C for 30 seconds was repeated 15 times. Thereafter, an extension reaction was carried out at 72°C for 7 minutes. A resulting DNA fragment of approximately 0.4 kbp was digested with a restriction enzyme, SpeI, and inserted into the expression vector pCEP4d-FLAG (approximately 7.7 kbp) which had been digested with XbaI, to obtain an expression vector pCEPdE2-FLAG. In the resulting expression vector pCEPdE2-FLAG, the XbaI recognition sequence, the NheI recognition sequence, the NotI recognition sequence, the BamHI recognition sequence, and the FLAG tag were arranged from the promoter to the downstream thereof.

### Example 2: Cloning of full-length ORF gene of novel protease gene MDTS9

A PCR was carried out, using a combination of the oligonucleotide consisting of the base sequence of SEQ ID NO: 7 (having an SpeI recognition sequence and a Kozak sequence added to the 5'-terminus) and the oligonucleotide consisting of the base sequence of SEQ ID NO: 8 (having an NotI recognition sequence added to the 5'-terminus) as the primers, a human fetal kidney cDNA library (Marathon-Ready^{™} cDNA; manufactured by Clontech) as a template, and DNA polymerase (TaKaRa LA Taq^{™}; manufactured by Takara-shuzo) as the DNA polymerase. In the PCR, a thermal denaturing reaction was first performed at 94°C for 2 minutes. Then, a cycle composed of treatments at 98°C for 10 seconds, and 68°C for 2 minutes and 30 seconds was repeated 40 times. Thereafter, an extension reaction was carried out at 68°C for 7 minutes. A resulting PCR product, a DNA fragment with approximately 2.2 kbp (having the SpeI recognition sequence and the Kozak sequence added to the 5'-terminus, and the NotI recognition sequence added to the 3'-terminus), was subcloned into a plasmid PCR2.1 (manufactured by Invitrogen) to obtain a clone pMDTS9Cys1.

The resulting plasmid pMDTS9Cysl was digested with restriction enzymes, SpeI and NotI, and a resulting DNA fragment of approximately 2.2 kbp was inserted into the XbaI and NotI site of the plasmid pCEPdE2-FLAG constructed in Example 1 to construct a plasmid pCEPdE2-MDTS9Cys1-FLAG.

A PCR was carried out, using a combination of the oligonucleotide consisting of the base sequence of SEQ ID NO: 9 (having a SpeI recognition sequence and a Kozak sequence added to the 5'-terminus) and the oligonucleotide consisting of the base sequence of SEQ ID NO: 10 as the primers, a human fetal kidney cDNA library (Marathon-Ready^{™} cDNA; manufactured by Clontech) as a template, and DNA polymerase (TaKaRa LA Taq^{™}; manufactured by Takara-shuzo) as the DNA polymerase. In the PCR, a thermal denaturing reaction was first performed at 94°C for 2 minutes. Then, a cycle composed of treatments at 98°C for 10 seconds, and 68°C for 30 seconds was repeated 45 times. Thereafter, an extension reaction was carried out at 68°C for 7 minutes. A resulting PCR product, a DNA fragment with approximately 0.2 kbp (having the SpeI recognition sequence and the Kozak sequence added to the 5'-terminus, and the NotI recognition sequence added to the 3'-terminus), was subcloned into a plasmid PCR2.1 (manufactured by Invitrogen) to obtain a clone pMDTS9(5S2-12).

A SpeI-NcoI DNA fragment A of approximately 0.2 kbp obtained by digesting the resulting plasmid pMDTS9(5S2-12) with restriction enzymes SpeI and NcoI, and a NcoI-NotI DNA fragment B of approximately 2.0 kbp obtained by digesting the previously resulting plasmid pMDTS9Cys1 with restriction enzymes NcoI and NotI, were inserted into the XbaI and NotI site of the pCEPdE2-FLAG constructed in Example 1 to construct a plasmid pCEPdE2-MDTS9Cys2-FLAG. Similarly, the DNA fragment A and the DNA fragment B were inserted into the SpeI and NotI site of a plasmid pZErO-2 (manufactured by Invitrogen) to construct a plasmid pZErO-MDTS9Cys2.

A PCR was carried out, using a combination of the oligonucleotide consisting of the base sequence of SEQ ID NO: 11 and the oligonucleotide consisting of the base sequence of SEQ ID NO: 12 as the primers, a human fetal kidney cDNA library (Marathon-Ready^{™} cDNA; manufactured by Clontech) as a template, and DNA polymerase (TaKaRa LA Taq^{™}; manufactured by Takara-shuzo) as the DNA polymerase. In the PCR, a thermal denaturing reaction was first performed at 94°C for 2 minutes. Then, a cycle composed of treatments at 98°C for 10 seconds, and 68°C for 2 minutes and 30 seconds was repeated 40 times. Thereafter, an extension reaction was carried out at 68°C for 7 minutes. A resulting PCR product, a DNA fragment with approximately 2.1 kbp was subcloned into a plasmid PCR2.1 (manufactured by Invitrogen) to obtain a clone pMDTS9-3H.

A DNA fragment of approximately 2.1 kbp generated by digesting the resulting plasmid pMDTS9-3H with restriction enzymes SphI and NotI was ligated into a DNA fragment of approximately 9.3 kbp generated by digesting the previously resulting plasmid pCEPdE2-MDTS9Cys2-FLAG with restriction enzymes SphI and NotI to construct a plasmid pCEPdE2-MDTS9Full-FLAG.

The resulting plasmid pCEPdE2-MDTS9Full-FLAG contains a gene consisting of the 1st to 3672nd bases in the base sequence of SEQ ID NO: 1, i.e., the base sequence of the novel protease gene MDTS9. A polypeptide having the 1st to 1224th amino acid sequence in the amino acid sequence of SEQ ID NO: 2 and the amino acid sequence of SEQ ID NO: 21 added to the C-terminus thereof can be expressed from an animal cell as a host.

Further, the previously resulting plasmid pCEPdE2-MDTS9Cys2-FLAG contains a gene consisting of the 1st to 2250th bases in the base sequence of SEQ ID NO: 1, i.e., the base sequence of the novel protease gene MDTS9. A polypeptide having the 1st to 750th amino acid sequence in the amino acid sequence of SEQ ID NO: 2 and the amino acid sequence of SEQ ID NO: 21 added to the C-terminus thereof can be expressed from an animal cell as a host. In this connection, it is considered that the polypeptide consisting of the amino acid sequence of SEQ IF NO: 2 is an ADAMTS protease.

### Example 3: Expression of MDTS9 truncated protein (MDTS9Cys2) and MDTS9 full-length protein (MDTS9Full)

A commercially available transfection reagent (FuGENE^{™}6 Transfection Reagent; manufactured by Boehringer Mannheim) was used, in accordance with a protocol attached thereto, to introduce the plasmid pCEPdE2-MDTS9Cys2-FLAG or plasmid pCEPdE2-MDTS9Full-FLAG prepared in Example 2, or the plasmid pCEPdE2-FLAG prepared in Example 1 as a control, into an HEK293-EBNA cell (manufactured by Invitrogen) cultured in a serum-containing medium [DMEM (GIBCO-BRL), 10% fetal bovine serum, 100 µg/mL penicillin, 100 µg/mL streptomycin, and 250 µg/mL G418 (manufactured by Nakarai Tesque, Inc.)].

After the plasmid introduction, cells were cultivated for 48 hours (hereinafter referred to as cultivation with serum). Alternatively, after the plasmid introduction, cells were cultivated for 16 hours, washed with PBS twice, and cultivated in a serum-free medium [DMEM (GIBCO-BRL), 100 µg/mL penicillin, 100 µg/mL streptomycin, and 250 µg/mL G418 (manufactured by Nakarai Tesque, Inc.)] for 32 hours (hereinafter referred to as cultivation without serum).

Each culture liquid obtained in the cultivation with serum or cultivation without serum was centrifuged at 3000 rpm for 10 minutes using a centrifuge (Type 8800; manufactured by Kubota Corporation) to obtain a culture supernatant. Each remaining cell after removing the culture medium, was treated with an extraction solution [20 mmol/L HEPES (pH7.4), 1% Triton X-100, 1% glycerol, and 0.1% bovine serum albumin (BSA)] for 15 minutes, and removed from a culture plate by pipetting. The resulting cell suspension was centrifuged at 3000 rpm for 10 minutes using a centrifuge (Type 8800; manufactured by Kubota Corporation) to separate a cell membrane bound fraction (supernatant) from a cell fraction (pellet).

The expression of the desired proteins in the resulting fractions (i.e., culture supernatant, cell membrane bound fraction, and cell fraction) was confirmed by a western blotting using an antibody (mouse anti-FLAG monoclonal antibody M2; manufactured by Sigma) against the FLAG tag added to the C-terminus. More particularly, each fraction was electrophoresed on an SDS/10%-20% acrylamide gel (manufactured by Daiichi Pure Chemicals) under reducing conditions using 2-ME, and transferred to a polyvinylidene difluoride (PVDF) membrane by a blotting apparatus. To the resulting PVDF membrane, a blocking agent (Block-ace manufactured by Dainippon Pharmaceutical) was added to perform a blocking. Then, the products on the membrane were reacted successively with the mouse anti-FLAG monoclonal antibody M2 and a rabbit anti-mouse IgG polyclonal antibody labeled with horseradish peroxidase (manufactured by Zymed or TAGO). Alternatively, after blocking, the products on the membrane were reacted successively with a biotinylated antibody M2 (manufactured by Sigma) and a streptoavidin labeled with horseradish peroxidase (manufactured by Amersham Pharmacia Biotech). After the reaction, an expression of the desired protein was confirmed by a commercially available western blotting detecting system (ECL Western Blotting Detecting System; manufactured by Amersham Pharmacia Biotech).

An apparent molecular weight on the SDS-polyacrylamide gel electrophoresis (SDS-PAGE) of the detected protein (i.e., MDTS9 truncated protein) in each fraction obtained by the cultivation without serum of the cell into which the plasmid pCEPdE2-MDTS9Cys2-FLAG was introduced, was approximately 55 to 65 kDa in the culture supernatant, approximately 55 to 65 kDa in the cell membrane bound fraction, and approximately 80 to 95 kDa in the cell fraction.

Further, the detected protein (i.e., MDTS9 full-length protein) in each fraction obtained by the cultivation without serum of the cell into which the plasmid pCEPdE2-MDTS9Full-FLAG was introduced, was mainly detected in the cell membrane bound fraction and the cell fraction. An apparent molecular weight on the SDS-PAGE thereof was approximately 130 to 140 kDa in all fractions.

### Example 4: Confirmation of protease activity of MDTS9 truncated protein

### (1) Construction of plasmid pCEPdE2-MDTS9Cys2E/Q-FLAG

A QuickChange^{™} Site-Directed Mutagenesis Kit (manufactured by Stratagene) was used, in accordance with a protocol attached thereto, to construct a plasmid pZErO-MDTS9Cys2E/Q containing a gene MDTS9Cys2E/Q in which Glu (glutamic acid) in His-Glu-Ser-Gly-His (SEQ ID NO: 22) was substituted with Gln (glutamine). The Glu is considered to be an active center. In this construction, the plasmid pZErO-MDTS9Cys2 prepared in Example 2 was used as a template, and the oligonucleotide consisting of the base sequence of SEQ ID NO: 13 and the oligonucleotide consisting of the base sequence of SEQ ID NO: 14 were used as a primer set.

A DNA fragment of approximately 2.3 kbp generated by digesting the resulting plasmid pZErO-MDTS9Cys2E/Q with restriction enzymes SpeI and NotI, was inserted into the XbaI and NotI site of the plasmid pCEPdE2-FLAG constructed in Example 1 to obtain a plasmid pCEPdE2-MDTS9Cys2E/Q-FLAG.

### (2) Confirmation of protease activity on the basis of complex formation with α₂-macroglobulin [0106]

Each culture supernatant (cultivation with serum) from each cell transfected with the plasmid pCEPdE2-MDTS9Cys2-FLAG prepared in Example 2 or the plasmid pCEPdE2-MDTS9Cys2E/Q-FLAG prepared in Example 4(1), or the plasmid pCEPdE2-FLAG prepared in Example 1 as a control, was electrophoresed (SDS-PAGE) under reducing conditions using 2-ME, and transferred to a PVDF membrane, as described in Example 3. To the resulting PVDF membrane, a blocking agent (Block-Ace manufactured by Dainippon Pharmaceutical) was added to perform a blocking. Then, the products on the membrane were reacted successively with the goat anti-α₂-macroglobulin antibody (manufactured by CEDARLANE) and a rabbit anti-goat IgG polyclonal antibody labeled with horseradish peroxidase (manufactured by Zymed Laboratories). After the reaction, an expression of the desired protein was confirmed by a commercially available western blotting detecting system (ECL Western Blotting Detecting System; manufactured by Amersham Pharmacia Biotech).

In the culture supernatant (cultivation with serum) from the cell transfected with the plasmid pCEPdE2-MDTS9Cys2-FLAG, a band of approximately 250 kDa was detected. The band was not detected in each culture supernatant (cultivation with serum) from the cell transfected with the plasmid pCEPdE2-MDTS9Cys2E/Q-FLAG or the plasmid pCEPdE2-FLAG. This result shows that the MDTS9 truncated protein (MDTS9Cys2) formed a complex with α₂-macroglobulin, and thus it was confirmed that the MDTS9 truncated protein (MDTS9Cys2) has a protease activity.

### Example 5: Confirmation of tissue distribution of MDTS9 gene expression

A tissue distribution of the MDTS9 gene expression was analyzed in accordance with the following procedures, using a commercially available cDNA panel [Human MTC Panel I (catalogue No. K1420-1), Human MTC Panel II (catalogue No. K1421-1), Human Fetal MTC Panel (catalogue No. K1425-1), and Human Tumor MTC Panel (catalogue No. K1422-1) in Multiple Tissue cDNA (MTC^{™}) Panel manufactured by Clontech].

More particularly, a PCR was carried out using a combination of the oligonucleotide consisting of the base sequence of SEQ ID NO: 15 and the oligonucleotide consisting of the base sequence of SEQ ID NO: 16 as primers, the cDNA panel as a template, and DNA polymerase (TaKaRa LA Taq^{™}; manufactured by Takara-shuzo). In the PCR, a thermal denaturing reaction was first performed at 94°C for 2 minutes. Then, a cycle composed of treatments at 98°C for 10 seconds, and 68°C for 1 minute and 30 seconds was repeated 44 times. Each reaction liquid was electrophoresed on an agarose gel to detect a DNA fragment of approximately 1.1 kbp derived from mRNA of the MDTS9 gene. As a result, it was revealed that mRNA of the MDTS9 gene was expressed in a kidney.

### Example 6: Induction of MDTS9 gene expression by TGF-β

### (1) Preparation of template cDNA

Normal human proximal tubule epithelial cells (5 x 10⁵ cells; manufactured by Clonetics) were seeded on a 6-well plate (manufactured by ASAHI TECHNOGLASS CORPORATION), and cultured for 1 day using Renal Epithelial Cell Medium kit (manufactured by Clonetics). The medium was changed to serum-free Renal Epithelial Cell Medium, and cultivation was further continued for 1 day. The medium was changed to serum-free Renal Epithelial Cell Medium containing 10 ng/mL (final concentration) of TGF-β1 (manufactured by Sigma), and the cells were cultured for 24 hours. In this connection, the medium of a control group was change to serum-free Renal Epithelial Cell Medium without TGF-β1, and the cells were cultured for 24 hours.

A total RNA was prepared from each treated group using a commercially available total RNA purifying reagent (ISOGEN; manufactured by Nippon Gene). The resulting total RNA was reacted with DNase (manufactured by Nippon Gene) at 37°C for 90 minutes. The DNase-treated total RNA (0.5 µg) was converted to cDNA by the Superscript first-strand system (for RT-PCR; manufactured by GIBCO-BRL).

### (2) Quantitative determination of MDTS9 mRNA by quantitative PCR

An analysis of an expression change in the normal human proximal tubule epithelial cell was carried out using the cDNA prepared in Example 6(1) as a template and a sequence detector (Prism7700 Sequence Detector; manufactured by Applied Biosystems). A combination of the oligonucleotide consisting of the base sequence of SEQ ID NO: 17 and the oligonucleotide consisting of the base sequence of SEQ ID NO: 18 were used as a primer set. A PCR was carried out, using a commercially available PCR reagent (SYBR Green PCR core reagent; manufactured by Applied Biosystems), by carrying out an initial denaturing reaction at 95°C for 10 minutes, and repeating a cycle reaction composed of treatments at 94°C for 15 seconds, 60°C for 30 seconds, and 72°C for 60 seconds 40 times.

In this connection, to calculate, as an internal standard, an amount of human β actin expressed, a PCR was carried out, using the above cDNA as a template and a combination of the oligonucleotide consisting of the base sequence of SEQ ID NO: 19 and the oligonucleotide consisting of the base sequence of SEQ ID NO: 20 as a primer set under the same conditions. Further, to obtain a standard curve for calculating an amount of mRNA expressed, a PCR was carried out, using the cDNA [prepared in Example 6(1)] from human proximal tubule epithelial cells without the stimulation by TGF-β1 as a template and the above primer set (i.e., a combination of the oligonucleotide consisting of the base sequence of SEQ ID NO: 17 and the oligonucleotide consisting of the base sequence of SEQ ID NO: 18, or a combination of the oligonucleotide consisting of the base sequence of SEQ ID NO: 19 and the oligonucleotide consisting of the base sequence of SEQ ID NO: 20) under the same conditions. An amount of the MDTS9 mRNA expressed in each condition was shown as a ratio to an amount of mRNA of β actin gene expressed in each condition to obtain an amount of the mRNA of the MDTS9 gene per a certain amount of the total RNA. As a result, it was revealed that TGF-β1 induced the gene expression of mRNA of the MDTS9 gene by approximately 8-fold.

### Example 7: Expression change of MDTS9 gene in rat renal failure model

### (1) Preparation of template cDNA

cDNA was prepared from a kidney of a rat 5/6 nephrectomy model [Kenjiro Kimura, "jin to toseki (kidney and dialysis)", 1991(suppl.), 431-439]. After 1, 2, 3, 4, 6, 8, and 10 weeks from 5/6 nephrectomy, five 5/6 nephrectomy rats and five sham operated rats were anatomized to remove kidneys. The kidneys were immediately frozen and kept at -80°C. The kidneys derived from each group were crushed using a cell crusher (CRYO-PRESS CP-100; manufactured by Microtec Nition) while being frozen by liquid nitrogen, and then a total RNA was prepared using a total RNA purifying reagent (ISOGEN; manufactured by Nippon Gene). The extracted total RNA was reacted with DNase (manufactured by Nippon Gene) at 37°C for 90 minutes. The DNase-treated total RNA (0.25 µg) was converted to cDNA by a Superscript first-strand system (for RT-PCR; manufactured by GIBCO-BRL).

### (2) Quantitative determination of mRNA of rat MDTS9 counterpart by quantitative PCR

An analysis of an expression change in a kidney of the rat renal failure model was carried out using the cDNA prepared in Example 7(1) as a template and a sequence detector (Prism7700 Sequence Detector; manufactured by Applied Biosystems). The oligonucleotide consisting of the base sequence of SEQ ID NO: 23 and the oligonucleotide consisting of the base sequence of SEQ ID NO: 24 were used as a primer set. A PCR was carried out, using a commercially available PCR reagent (SYBR Green PCR core reagent; manufactured by Applied Biosystems), by carrying out an initial denaturing reaction at 95°C for 10 minutes, and repeating a cycle reaction composed of treatments at 94°C for 15 seconds, 60°C for 30 seconds, and 72°C for 60 seconds 45 times.

To calculate, as an internal standard, an amount of human glyceraldehyde-3-phosphate dehydrogenase (G3PDH) expressed, a PCR was carried out, using the above cDNA as a template and the oligonucleotide consisting of the base sequence of SEQ ID NO: 25 and the oligonucleotide consisting of the base sequence of SEQ ID NO: 26 as a primer set under the same conditions. Further, to obtain a standard curve for calculating an amount of mRNA expressed, a PCR was carried out, using rat genomic DNA (manufactured by Clontech) as a template and the above primer set under the same conditions. An amount of the mRNA of the rat MDTS9 gene expressed in each condition was shown as a ratio to an amount of mRNA of G3PDH gene expressed in each condition to compare an amount of the mRNA of the rat MDTS9 gene per a certain amount of the total RNA in each group. As a result, it was found that the mRNA of the rat MDTS9 gene was expressed in the 5/6 nephrectomy rat by approximately 5-fold in comparison with the sham operated rat after 1 week from the operation, and that it was expressed by approximately 2-fold after 3 weeks (an amount of proteins in urine remarkably increased), 6 weeks (a kidney weight began to increase in comparison with a normal kidney weight), and 8 weeks (symptoms were aggravated) from the operation.

It was revealed from this Example that an expression of the MDTS9 gene is induced in the renal failure model.

### Example 8: Immunohistochemical staining of human renal tissue section

### (1) Preparation of anti-human MDTS9 antibody

A fusion protein (GST-MDTS9A) of a peptide consisting of the 280th to 410th amino acids in the amino acid sequence of SEQ ID NO: 2 and glutathion S-transferase (GST) was prepared, using a plasmid pGEX-6P-1 (manufactured by Amersham Pharmacia Biotech) as an expression vector and E. coli, in an inclusion body fraction, in accordance with a laboratory manual [Masato Okada and Kaoru Miyazaki, "Kaitei, Tanpakushitsu Jikken Noto, Jyo (Revision, Notebook for Protein Experiments)", Yodo-sha, p.162-179]. A preparative SDS-polyacrylamide gel electrophoresis (PAGE) was carried out using the inclusion body fraction, and then the desired GST-MDTS9A protein was extracted from the gel by a diffusion method [Masato Okada and Kaoru Miyazaki, "Kaitei, Tanpakushitsu Jikken Noto, Ge (Revision, Notebook for Protein Experiments)", Yodo-sha, p.48-51].

A rabbit (Japanese white) was immunized by the resulting GST-MDTS9A protein 5 times in total at an interval of 10 to 14 days to obtain an antiserum. An IgG fraction was purified from the antiserum by an affinity chromatography using a protein G Sepharose FF column (manufactured by Amersham Pharmacia Biotech). Then, an anti-human MDTS9 antibody was purified from the IgG fraction by an affinity chromatography using a column (MBP-MDTS9A column) in which a fusion protein (MBP-MDTS9A) of a peptide consisting of the 280th to 410th amino acids in the amino acid sequence of SEQ ID NO: 2 and mannose binding protein (MBP) was immobilized. The affinity purification by the protein G Sepharose FF column, immobilization of MBP-MDTS9A to a CNBr-activated Sepharose FF column (manufactured by Amersham Pharmacia Biotech), and the affinity purification by the MBP-MDTS9A column were carried out in accordance with protocols attached thereto. Further, a preparation of MBP-MDTS9A in E. coli and purification thereof were carried out using pMAL-c2E (manufactured by New England Biolabs) as an expression vector in accordance with an instruction "pMAL protein fusion and purification system" published thereby.

### (2) Detection of MDTS9 protein in human kidney

The anti-human MDTS9 antibody prepared in Example 8(1) was reacted to a tissue section which had been fixed by formalin and embedded with paraffin on a slide glass. Then, the tissue section was stained using a commercially available staining kit (VECTORSTAIN ABC-AP kit, catalog No. AK-5000; manufactured by VECTOR LABORATORIES) in accordance with a protocol attached thereto. In this procedure, an anti-rabbit antibody labeled with biotin (catalog No. BA-1000; manufactured by Vector) as a second antibody and an alkaline phosphatase substrate kit I (catalog No. SK-5100; manufactured by Vector) as a color-developing substrate were used. As a result, staining was observed in epithelial cells (particularly podocytes) of kidneys from a healthy person and a patient suffering from diabetic nephropathy (early stage or late stage).

It is apparent from this Example that the MDTS9 protein is expressed in human kidney.

### INDUSTRIAL APPLICABILITY

The polypeptide of the present invention is a novel protease, which is expressed in a kidney, induced by TGF-β, and involved with a metabolism in an extracellular matrix. Therefore, a substance inhibiting the protease activity of the polypeptide of the present invention is useful as an agent for treating chronic renal failure in which a long-term administration is foreseen, because it is highly possible that the substance suppresses or inhibits only a portion which is involved with a qualitative change and a quantitative increase of extracellular matrix components, among physiological actions of TGF-β. That is, according to the polypeptide of the present invention, a convenient screening system for an agent for treating chronic renal failure. Further, the polynucleotide, the expression vector, the cell, and the antibody of the present invention are useful for producing the polypeptide of the present invention.

### FREE TEXT IN SEQUENCE LISTING

Features of "Artificial Sequence" are described in the numeric identifier <223> in the Sequence Listing. More particularly, each of the base sequences of SEQ ID NOS: 3 and 4 is an artificially synthesized linker sequence. Each of the base sequences of SEQ ID NOS: 5-9 and 12-14 is an artificially synthesized primer sequence. The base sequence of SEQ ID NO: 21 is an amino acid sequence obtained by an expression of DNA containing a restriction enzyme NotI recognition nucleotide sequence and a nucleotide sequence encoding a FLAG tag amino acid sequence.

## Claims

1. A polypeptide exhibiting a protease activity and comprising (1) the amino acid sequence consisting of 1^{st} to 750th amino acids in the amino acid sequence of SEQ ID No: 2, (2) an amino acid sequence in which 1 to 10 amino acids are deleted, substituted, and/or inserted in the amino acid sequence consisting of 1^{st} to 750^{th} amino acids in the amino acid sequence of SEQ ID No: 2, or (3) an amino acid sequence in which 1 to 10 amino acids are deleted, substituted, and/or inserted in the amino acid sequence consisting of 1^{st} to 1224^{th} amino acids in the amino acid sequence of SEQ ID No: 2.

2. The polypeptide of claim 1 comprising the amino acid sequence consisting of 1^{st} to 750^{th} amino acids in the amino acid sequence of SEQ ID No: 2.

3. The polypeptide of claim 1 consisting of (1) an amino acid sequence in which 1 to 10 amino acids are deleted, substituted, and/or inserted in the amino acid sequence consisting of 1^{st} to 750^{th} amino acids in the amino acid sequence of SEQ ID No: 2, or (2) an amino acid sequence in which 1 to 10 amino acids are deleted, substituted, and/or inserted in the amino acid sequence consisting of 1^{st} to 1224^{th} amino acids in the amino acid sequence of SEQ ID No: 2.

4. The polypeptide of claim 1 consisting of (1) 1^{st} to 750^{th} amino acids in the amino acid sequence of SEQ ID No: 2, or (2) 1^{st} to 1224^{th} amino acids in the amino acid sequence of SEQ ID No: 2.

5. A polynucleotide encoding the polypeptide according to any one of claims 1 to 4.

6. An expression vector comprising the polynucleotide according to claim 5.

7. A cell transfected with the expression vector according to claim 6.

8. An antibody or a fragment thereof, which binds to the polypeptide according to any one of claims 1 to 4.

9. A process for producing the polypeptide according to any one of claims 1 to 4, comprising the steps of:
culturing the cell according to claim 7, and
recovering the polypeptide according to any one of claims 1 to 4.

10. A method for detecting whether or not a compound to be tested inhibits a protease activity of the polypeptide according to any one of claims 1 to 4, comprising the steps of:
bringing into contact (1) said polypeptide, (2) α₂-macroglobulin, and (3) said compound to be tested, and analysing whether or not said polypeptide and α₂-macroglobulin form a complex which is not dissociated by SDS and/or a reducing agent.

11. The method according to claim 10, further comprising the step of:
selecting a substance inhibiting the protease activity.

12. A method for screening a substance for treating chronic renal failure by the method according to claim 11.

## Patentansprüche

1. Polypeptid, das eine Proteaseaktivität zeigt und umfassend (1) die Aminosäuresequenz bestehend aus der 1sten bis 750sten Aminosäure in der Aminosäuresequenz von SEQ ID NO: 2, (2) eine Aminosäuresequenz, worin 1 bis 10 Aminosäuren in der Aminosäuresequenz bestehend aus der 1sten bis 750sten Aminosäure der Aminosäuresequenz von SEQ ID NO: 2 deletiert, substituiert und/oder inseriert sind, oder (3) eine Aminosäuresequenz, worin 1 bis 10 Aminosäuren in der Aminosäuresequenz bestehend aus der 1sten bis 1224sten Aminosäure in der Aminosäuresequenz von SEQ ID NO: 2 deletiert, substituiert und/oder inseriert sind.

2. Polypeptid gemäß Anspruch 1, umfassend die Aminosäuresequenz bestehend aus der 1sten bis 750sten Aminosäure in der Aminosäuresequenz von SEQ ID NO: 2.

3. Polypeptid gemäß Anspruch 1, bestehend aus (1) einer Aminosäuresequenz, worin 1 bis 10 Aminosäuren in der Aminosäuresequenz bestehend aus der 1sten bis 750sten Aminosäure in der Aminosäuresequenz von SEQ ID NO: 2 deletiert, substituiert und/oder inseriert sind, oder (2) einer Aminosäuresequenz, worin 1 bis 10 Aminosäuren in der Aminosäuresequenz bestehend aus der 1sten bis 1224sten Aminosäure in der Aminosäuresequenz von SEQ ID NO: 2 deletiert, substituiert und/oder inseriert sind.

4. Polypeptid gemäß Anspruch 1, bestehend aus (1) den 1sten bis 750sten Aminosäuren der Aminosäuresequenz von SEQ ID NO: 2 oder (2) den 1sten bis 1224sten Aminosäuren der Aminosäuresequenz von SEQ ID NO: 2.

5. Polynukleotid, codierend das Polypeptid gemäß einem der Ansprüche 1 bis 4.

6. Expressionsvektor, umfassend das Polynukleotid gemäß Anspruch 5.

7. Zelle, transfiziert mit dem Expressionsvektor gemäß Anspruch 6.

8. Antikörper oder Fragment davon, der an das Polypeptid gemäß einem der Ansprüche 1 bis 4 bindet.

9. Verfahren zur Erzeugung des Polypeptids gemäß einem der Ansprüche 1 bis 4, umfassend die Schritte der Kultivierung der Zelle gemäß Anspruch 7 und der Gewinnung des Polypeptids gemäß einem der Ansprüche 1 bis 4.

10. Verfahren zum Nachweis, ob oder nicht eine zu testende Verbindung eine Proteaseaktivität des Polypeptids gemäß einem der Ansprüche 1 bis 4 inhibiert, umfassend die folgenden Schritte:
Kontaktieren von (1) dem Polypeptid, (2) α₂-Makroglobulin und (3) der zu testenden Verbindung und Analyse, ob oder nicht das Polypeptid und
α₂-Makroglobulin einen Komplex bilden, der durch SDS und/oder ein Reduktionsmittel nicht dissoziiert wird.

11. Verfahren gemäß Anspruch 10, weiterhin umfassend den Schritt der Selektion einer Substanz, die die Proteaseaktivität inhibiert.

12. Verfahren zum Screening nach einer Substanz zur Behandlung von chronischem Nierenversagen durch das Verfahren gemäß Anspruch 11.

## Revendications

1. Polypeptide présentant une activité protéase et comprenant (1) la séquence d'acides aminés consistant en du 1^{er} au 750^{ème} acide aminé se trouvant dans la séquence d'acides aminés de la SEQ ID No : 2, (2) une séquence d'acides aminés dans laquelle de 1 à 10 acides aminés sont supprimés, substitués, et/ou insérés dans la séquence d'acides aminés consistant en du 1^{er} au 750^{ème} acide aminé se trouvant dans la séquence d'acides aminés de la SEQ ID No : 2, ou (3) une séquence d'acides aminés dans laquelle de 1 à 10 acides aminés sont supprimés, substitués, et/ou insérés dans la séquence d'acides aminés consistant en du 1^{er} au 1224^{ème} acide aminé se trouvant dans la séquence d'acides aminés de la SEQ ID No : 2.

2. Polypeptide selon la revendication 1 comprenant la séquence d'acides aminés consistant en du 1^{er} au 750^{ème} acide aminé se trouvant dans la séquence d'acides aminés de la SEQ ID No : 2.

3. Polypeptide selon la revendication 1 consistant en (1) une séquence d'acides aminés dans laquelle de 1 à 10 acides aminés sont supprimés, substitués, et/ou insérés dans la séquence d'acides aminés consistant en du 1^{er} au 750^{ème} acide aminé se trouvant dans la séquence d'acides aminés de la SEQ ID No : 2, ou (2) une séquence d'acides aminés dans laquelle de 1 à 10 acides aminés sont supprimés, substitués, et/ou insérés dans la séquence d'acides aminés consistant en du 1^{er} au 1224^{ème} acide aminé se trouvant dans la séquence d'acides aminés de la SEQ ID No : 2.

4. Polypeptide selon la revendication 1 consistant en (1) le 1^{er} au 750^{ème} acide aminé se trouvant dans la séquence d'acides aminés de la SEQ ID No : 2, ou (2) le 1^{er} au 1224^{ème} acide aminé se trouvant dans la séquence d'acides aminés de la SEQ ID No : 2.

5. Polynucléotide codant pour le polypeptide selon l'une quelconque des revendications 1 à 4.

6. Vecteur d'expression comprenant le polynucléotide selon la revendication 5.

7. Cellule transfectée avec le vecteur d'expression selon la revendication 6.

8. Anticorps ou fragment de celui-ci, qui se lie au polypeptide selon l'une quelconque des revendications 1 à 4.

9. Processus destiné à produire le polypeptide selon l'une quelconque des revendications 1 à 4, comprenant les étapes :
de cultiver la cellule selon la revendication 7, et
de récupérer le polypeptide selon l'une quelconque des revendications 1 à 4.

10. Procédé destiné à détecter si oui ou non un composé à tester inhibe une activité protéase du polypeptide selon l'une quelconque des revendications 1 à 4, comprenant les étapes :
d'amener en contact (1) ledit polypeptide, (2) une α₂-macroglobuline, et (3) ledit composé à tester, et
d'analyser si oui ou non ledit polypeptide et l'α₂-macroglobuline forment un complexe qui n'est pas dissocié par du SDS et/ou un agent réducteur.

11. Procédé selon la revendication 10, comprenant en outre l'étape :
de choisir une substance inhibant l'activité protéase.

12. Procédé de criblage d'une substance destinée à traiter une insuffisance rénale chronique par le procédé selon la revendication 11.


### SEQUENCE LISTING

<110> Yamanouchi Pharmaceutical Co., Ltd.
<120> Novel protease
<130> Y0132PCT-664
<150> JP 2000-393372
   <151> 2000-12-25
<160> 26
<210> 1
   <211> 3675
   <212> DNA
   <213> Homo sapiens
<220>
   <221> CDS
   <222> (1).. (3675)
<400> 1
   atg aag ccc cgc gcg cgc gga tgg cgg ggc ttg gcg gcg ctg tgg atg      48
   Met Lys Pro Arg Ala Arg Gly Trp Arg Gly Leu Ala Ala Leu Trp Met
    1               5                  10                  15
   ctg ttg gcg cag gtg gcc gag cag gca cct gcg tgc gcc atg gga ccc      96
   Leu Leu Ala Gln Val Ala Glu Gln Ala Pro Ala Cys Ala Met Gly Pro
               20                  25                  30
   gca gcg gca gcg cct ggg agc ccg agc gtc ccg cgt cct cct cca ccc     144
   Ala Ala Ala Ala Pro Gly Ser Pro Ser Val Pro Arg Pro Pro Pro Pro
           35                  40                  45
   gcg gag cgg ccg ggc tgg atg gaa aag ggc gaa tat gac ctg gtc tct      192
   Ala Glu Arg Pro Gly Trp Met Glu Lys Gly Glu Tyr Asp Leu Val Ser
       50                  55                  60
   gcc tac gag gtt gac cac agg ggc gat tac gtg tcc cat gaa atc atg      240
   Ala Tyr Glu Val Asp His Arg Gly Asp Tyr Val Ser His Glu Ile Met
   65                  70                  75                  80
   cac cat cag cgg cgg aga aga gca gtg gcc gtg tcc gag gtt gag tct     288
   His His Gln Arg Arg Arg Arg Ala Val Ala Val Ser Glu Val Glu Ser
                   85                  90                  95
   ctt cac ctt cgg ctg aaa ggc tcc agg cac gac ttc cac gtg gat ctg      336
   Leu His Leu Arg Leu Lys Gly Ser Arg His Asp Phe His Val Asp Leu
               100                 105                 110
   agg act tcc agc agc cta gtg gct cct ggc ttt att gtg cag acg ttg      384
   Arg Thr Ser Ser Ser Leu Val Ala Pro Gly Phe Ile Val Gln Thr Leu
           115                 120                 125
   gga aag aca ggc act aag tct gtg cag act tta ccg cca gag gac ttc      432
   Gly Lys Thr Gly Thr Lys Ser Val Gln Thr Leu Pro Pro Glu Asp Phe
       130                 135                 140
   tgt ttc tat caa ggc tct ttg cga tca cac aga aac tcc tca gtg gcc      480
   Cys Phe Tyr Gln Gly Ser Leu Arg Ser His Arg Asn Ser Ser Val Ala
   145                 150                 155                 160
   ctt tca acc tgc caa ggc ttg tca ggc atg ata cga aca gaa gag gca      528
   Leu Ser Thr Cys Gln Gly Leu Ser Gly Met Ile Arg Thr Glu Glu Ala
                   165                 170                 175
   gat tac ttc cta agg cca ctt cct tca cac ctc tca tgg aaa ctc ggc      576
   Asp Tyr Phe Leu Arg Pro Leu Pro Ser His Leu Ser Trp Lys Leu Gly
               180                 185                 190
   aga gct gcc caa ggc agc tcg cca tcc cac gta ctg tac aag aga tcc      624
   Arg Ala Ala Gln Gly Ser Ser Pro Ser His Val Leu Tyr Lys Arg Ser
           195                 200                 205
   aca gag ccc cat gct cct ggg gcc agt gag gtc ctg gtg acc tca agg      672
   Thr Glu Pro His Ala Pro Gly Ala Ser Glu Val Leu Val Thr Ser Arg
       210                 215                 220
   aca tgg gag ctg gca cat caa ccc ctg cac agc agc gac ctt cgc ctg      720
   Thr Trp Glu Leu Ala His Gln Pro Leu His Ser Ser Asp Leu Arg Leu
   225                 230                 235                 240
   gga ctg cca caa aag cag cat ttc tgt gga aga cgc aag aaa tac atg      768
   Gly Leu Pro Gln Lys Gln His Phe Cys Gly Arg Arg Lys Lys Tyr Met
                   245                 250                 255
   ccc cag cct ccc aag gaa gac ctc ttc atc ttg cca gat gag tat aag      816
   Pro Gln Pro Pro Lys Glu Asp Leu Phe Ile Leu Pro Asp Glu Tyr Lys
               260                 265                 270
   tct tgc tta cgg cat aag cgc tct ctt ctg agg tcc cat aga aat gaa      864
   Ser Cys Leu Arg His Lys Arg Ser Leu Leu Arg Ser His Arg Asn Glu
           275                 280                 285
   gaa ctg aac gtg gag acc ttg gtg gtg gtc gac aaa aag atg atg caa     912
   Glu Leu Asn Val Glu Thr Leu Val Val Val Asp Lys Lys Met Met Gln
       290                 295                 300
   aac cat ggc cat gaa aat atc acc acc tac gtg ctc acg ata ctc aac     960
   Asn His Gly His Glu Asn Ile Thr Thr Tyr Val Leu Thr Ile Leu Asn
   305                 310                 315                 320
   atg gta tct gct tta ttc aaa gat gga aca ata gga gga aac atc aac    1008
   Met Val Ser Ala Leu Phe Lys Asp Gly Thr Ile Gly Gly Asn Ile Asn
                   325                 330                 335
   att gca att gta ggt ctg att ctt cta gaa gat gaa cag cca gga ctg    1056
   Ile Ala Ile Val Gly Leu Ile Leu Leu Glu Asp Glu Gln Pro Gly Leu
               340                 345                 350
   gtg ata agt cac cac gca gac cac acc tta agt agc ttc tgc cag tgg    1104
   Val Ile Ser His His Ala Asp His Thr Leu Ser Ser Phe Cys Gln Trp
           355                 360                 365
   cag tct gga ttg atg ggg aaa gat ggg act cgt cat gac cac gcc atc    1152
   Gln Ser Gly Leu Met Gly Lys Asp Gly Thr Arg His Asp His Ala Ile
       370                 375                 380
   tta ctg act ggt ctg gat ata tgt tcc tgg aag aat gag ccc tgt gac    1200
   Leu Leu Thr Gly Leu Asp Ile Cys Ser Trp Lys Asn Glu Pro Cys Asp
   385                 390                 395                 400
   act ttg gga ttt gca ccc ata agt gga atg tgt agt aaa tat cgc agc    1248
   Thr Leu Gly Phe Ala Pro Ile Ser Gly Met Cys Ser Lys Tyr Arg Ser
                   405                 410                 415
   tgc acg att aat gaa gat aca ggt ctt gga ctg gcc ttc acc att gcc    1296
   Cys Thr Ile Asn Glu Asp Thr Gly Leu Gly Leu Ala Phe Thr Ile Ala
               420                 425                 430
   cat gag tct gga cac aac ttt ggc atg att cat gat gga gaa ggg aac    1344
   His Glu Ser Gly His Asn Phe Gly Met Ile His Asp Gly Glu Gly Asn
           435                 440                 445
   atg tgt aaa aag tcc gag ggc aac atc atg tcc cct aca ttg gca gga    1392
   Met Cys Lys Lys Ser Glu Gly Asn Ile Met Ser Pro Thr Leu Ala Gly
       450                 455                 460
   cgc aat gga gtc ttc tcc tgg tca ccc tgc agc cgc cag tat cta cac 1440
   Arg Asn Gly Val Phe Ser Trp Ser Pro Cys Ser Arg Gln Tyr Leu His
   465 470 475 480
   aaa ttt cta agc acc gct caa gct atc tgc ctt gct gat cag cca aag 1488
   Lys Phe Leu Ser Thr Ala Gln Ala Ile Cys Leu Ala Asp Gln Pro Lys
   485 490 495
   cct gtg aag gaa tac aag tat cct gag aaa ttg cca gga gaa tta tat 1536
   Pro Val Lys Glu Tyr Lys Tyr Pro Glu Lys Leu Pro Gly Glu Leu Tyr
   500 505 510
   gat gca aac aca cag tgc aag tgg cag ttc gga gag aaa gcc aag ctc 1584
   Asp Ala Asn Thr Gln Cys Lys Trp Gln Phe Gly Glu Lys Ala Lys Leu
   515 520 525
   tgc atg ctg gac ttt aaa aag gac atc tgt aaa gcc ctg tgg tgc cat 1632
   Cys Met Leu Asp Phe Lys Lys Asp Ile Cys Lys Ala Leu Trp Cys His
   530 535 540
   cgt att gga agg aaa tgt gag act aaa ttt atg cca gca gca gaa ggc 1680
   Arg Ile Gly Arg Lys Cys Glu Thr Lys Phe Met Pro Ala Ala Glu Gly
   545 550 555 560
   aca att tgt ggg cat gac atg tgg tgc cgg gga gga cag tgt gtg aaa 1728
   Thr Ile Cys Gly His Asp Met Trp Cys Arg Gly Gly Gln Cys Val Lys
   565 570 575
   tat ggt gat gaa ggc ccc aag ccc acc cat ggc cac tgg tcg gac tgg 1776
   Tyr Gly Asp Glu Gly Pro Lys Pro Thr His Gly His Trp Ser Asp Trp
   580 585 590
   tct tct tgg tcc cca tgc tcc agg acc tgc gga ggg gga gta tct cat 1824
   Ser Ser Trp Ser Pro Cys Ser Arg Thr Cys Gly Gly Gly Val Ser His
   595 600 605
   agg agt cgc ctc tgc acc aac ccc aag cca tcg cat gga ggg aag ttc 1872
   Arg Ser Arg Leu Cys Thr Asn Pro Lys Pro Ser His Gly Gly Lys Phe
   610 615 620
   tgt gag ggc tcc act cgc act ctg aag ctc tgc aac agt cag aaa tgt 1920
   Cys Glu Gly Ser Thr Arg Thr Leu Lys Leu Cys Asn Ser Gln Lys Cys
   625 630 635 640
   ccc cgg gac agt gtt gac ttc cgt gct gct cag tgt gcc gag cac aac 1968
   Pro Arg Asp Ser Val Asp Phe Arg Ala Ala Gln Cys Ala Glu His Asn
   645 650 655
   agc aga cga ttc aga ggg cgg cac tac aag tgg aag cct tac act caa 2016
   Ser Arg Arg Phe Arg Gly Arg His Tyr Lys Trp Lys Pro Tyr Thr Gln
   660 665 670
   gta gaa gat cag gac tta tgc aaa ctc tac tgt atc gca gaa gga ttt 2064
   Val Glu Asp Gln Asp Leu Cys Lys Leu Tyr Cys Ile Ala Glu Gly Phe
   675 680 685
   gat ttc ttc ttt tct ttg tca aat aaa gtc aaa gat ggg act cca tgc 2112
   Asp Phe Phe Phe Ser Leu Ser Asn Lys Val Lys Asp Gly Thr Pro Cys
   690 695 700
   tcg gag gat agc cgt aat gtt tgt ata gat ggg ata tgt gag aga gtt 2160
   Ser Glu Asp Ser Arg Asn Val Cys Ile Asp Gly Ile Cys Glu Arg Val
   705 710 715 720
   gga tgt gac aat gtc ctt gga tct gat gct gtt gaa gac gtc tgt ggg 2208
   Gly Cys Asp Asn Val Leu Gly Ser Asp Ala Val Glu Asp Val Cys Gly
   725 730 735
   gtg tgt aac ggg aat aac tca gcc tgc acg att cac agg ggt ctc tac 2256
   Val Cys Asn Gly Asn Asn Ser Ala Cys Thr Ile His Arg Gly Leu Tyr
   740 745 750
   acc aag cac cac cac acc aac cag tat tat cac atg gtc acc att cct 2304
   Thr Lys His His His Thr Asn Gln Tyr Tyr His Met Val Thr Ile Pro
   755 760 765
   tct gga gcc cgg agt atc cgc atc tat gaa atg aac gtc tct acc tcc 2352
   Ser Gly Ala Arg Ser Ile Arg Ile Tyr Glu Met Asn Val Ser Thr Ser
   770 775 780
   tac att tct gtg cgc aat gcc ctc aga agg tac tac ctg aat ggg cac 2400
   Tyr Ile Ser Val Arg Asn Ala Leu Arg Arg Tyr Tyr Leu Asn Gly His
   785 790 795 800
   tgg acc gtg gac tgg ccc ggc cgg tac aaa ttt tcg ggc act act ttc 2448
   Trp Thr Val Asp Trp Pro Gly Arg Tyr Lys Phe Ser Gly Thr Thr Phe
   805 810 815
   gac tac aga cgg tcc tat aat gag ccc gag aac tta atc gct act gga 2496
   Asp Tyr Arg Arg Ser Tyr Asn Glu Pro Glu Asn Leu Ile Ala Thr Gly
   820 825 830
   cca acc aac gag aca ctg att gtg gag ctg ctg ttt cag gga agg aac 2544
   Pro Thr Asn Glu Thr Leu Ile Val Glu Leu Leu Phe Gln Gly Arg Asn
   835 840 845
   ccg ggt gtt gcc tgg gaa tac tcc atg cct cgc ttg ggg acc gag aag 2592
   Pro Gly Val Ala Trp Glu Tyr Ser Met Pro Arg Leu Gly Thr Glu Lys
   850 855 860
   cag ccc cct gcc cag ccc agc tac act tgg gcc atc gtg cgc tct gag 2640
   Gln Pro Pro Ala Gln Pro Ser Tyr Thr Trp Ala Ile Val Arg Ser Glu
   865 870 875 880
   tgc tcc gtg tcc tgc gga ggg gga cag atg acc gtg aga gag ggc tgc 2688
   Cys Ser Val Ser Cys Gly Gly Gly Gln Met Thr Val Arg Glu Gly Cys
   885 890 895
   tac aga gac ctg aag ttt caa gta aat atg tcc ttc tgc aat ccc aag 2736
   Tyr Arg Asp Leu Lys Phe Gln Val Asn Met Ser Phe Cys Asn Pro Lys
   900 905 910
   aca cga cct gtc acg ggg ctg gtg cct tgc aaa gta tct gcc tgt cct 2784
   Thr Arg Pro Val Thr Gly Leu Val Pro Cys Lys Val Ser Ala Cys Pro
   915 920 925
   ccc agc tgg tcc gtg ggg aac tgg agt gcc tgc agt cgg acg tgt ggc 2832
   Pro Ser Trp Ser Val Gly Asn Trp Ser Ala Cys Ser Arg Thr Cys Gly
   930 935 940
   ggg ggt gcc cag agc cgc ccc gtg cag tgc aca cgg cgg gtg cac tat 2880
   Gly Gly Ala Gln Ser Arg Pro Val Gln Cys Thr Arg Arg Val His Tyr
   945 950 955 960
   gac tcg gag cca gtc ccg gcc agc ctg tgc cct cag cct gct ccc tcc 2928
   Asp Ser Glu Pro Val Pro Ala Ser Leu Cys Pro Gln Pro Ala Pro Ser
   965 970 975
   agc agg cag gcc tgc aac tct cag agc tgc cca cct gca tgg agc gcc 2976
   Ser Arg Gln Ala Cys Asn Ser Gln Ser Cys Pro Pro Ala Trp Ser Ala
   980 985 990
   ggg ccc tgg gca gag tgc tca cac acc tgt ggg aag ggg tgg agg aag 3024
   Gly Pro Trp Ala Glu Cys Ser His Thr Cys Gly Lys Gly Trp Arg Lys
   995 1000 1005
   cgg gca gtg gcc tgt aag agc acc aac ccc tcg gcc aga gcg cag ctg 3072
   Arg Ala Val Ala Cys Lys Ser Thr Asn Pro Ser Ala Arg Ala Gln Leu
   1010 1015 1020
   ctg ccc gac gct gtc tgc acc tcc gag ccc aag ccc agg atg cat gaa 3120
   Leu Pro Asp Ala Val Cys Thr Ser Glu Pro Lys Pro Arg Met His Glu
   1025 1030 1035 1040
   gcc tgt ctg ctt cag cgc tgc cac aag ccc aag aag ctg cag tgg ctg 3168
   Ala Cys Leu Leu Gln Arg Cys His Lys Pro Lys Lys Leu Gln Trp Leu
   1045 1050 1055
   gtg tcc gcc tgg tcc cag tgc tct gtg aca tgt gaa aga gga aca cag 3216
   Val Ser Ala Trp Ser Gln Cys Ser Val Thr Cys Glu Arg Gly Thr Gln
   1060 1065 1070
   aaa aga ttc tta aaa tgt gct gaa aag tat gtt tct gga aag tat cga 3264
   Lys Arg Phe Leu Lys Cys Ala Glu Lys Tyr Val Ser Gly Lys Tyr Arg
   1075 1080 1085
   gag ctg gcc tca aag aag tgc tca cat ttg ccg aag ccc agc ctg gag 3312
   Glu Leu Ala Ser Lys Lys Cys Ser His Leu Pro Lys Pro Ser Leu Glu
   1090 1095 1100
   ctg gaa cgt gcc tgc gcc ccg ctt cca tgc ccc agg cac ccc cca ttt 3360
   Leu Glu Arg Ala Cys Ala Pro Leu Pro Cys Pro Arg His Pro Pro Phe
   1105 1110 1115 1120
   gct gct gcg gga ccc tcg agg ggc agc tgg ttt gcc tca ccc tgg tct 3408
   Ala Ala Ala Gly Pro Ser Arg Gly Ser Trp Phe Ala Ser Pro Trp Ser
   1125 1130 1135
   cag tgc acg gcc agc tgt ggg gga ggc gtt cag acg agg tcc gtg cag 3456
   Gln Cys Thr Ala Ser Cys Gly Gly Gly Val Gln Thr Arg Ser Val Gln
   1140 1145 1150
   tgc ctg gct ggg ggc cgg ccg gcc tca ggc tgc ctc ctg cac cag aag 3504
   Cys Leu Ala Gly Gly Arg Pro Ala Ser Gly Cys Leu Leu His Gln Lys
   1155 1160 1165
   cct tcg gcc tcc ctg gcc tgc aac act cac ttc tgc ccc att gca gag 3552
   Pro Ser Ala Ser Leu Ala Cys Asn Thr His Phe Cys Pro Ile Ala Glu
   1170 1175 1180
   aag aaa gat gcc ttc tgc aaa gac tac ttc cac tgg tgc tac ctg gta 3600
   Lys Lys Asp Ala Phe Cys Lys Asp Tyr Phe His Trp Cys Tyr Leu Val
   1185 1190 1195 1200
   ccc cag cac ggg atg tgc agc cac aag ttc tac ggc aag cag tgc tgc 3648
   Pro Gln His Gly Met Cys Ser His Lys Phe Tyr Gly Lys Gln Cys Cys
   1205 1210 1215
   aag act tgc tct aag tcc aac ttg tga 3675
   Lys Thr Cys Ser Lys Ser Asn Leu
   1220 1225
<210> 2
   <211> 1224
   <212> PRT
   <213> Homo sapiens
<400> 2
   Met Lys Pro Arg Ala Arg Gly Trp Arg Gly Leu Ala Ala Leu Trp Met
   1 5 10 15
   Leu Leu Ala Gln Val Ala Glu Gln Ala Pro Ala Cys Ala Met Gly Pro
   20 25 30
   Ala Ala Ala Ala Pro Gly Ser Pro Ser Val Pro Arg Pro Pro Pro Pro
   35 40 45
   Ala Glu Arg Pro Gly Trp Met Glu Lys Gly Glu Tyr Asp Leu Val Ser
   50 55 60
   Ala Tyr Glu Val Asp His Arg Gly Asp Tyr Val Ser His Glu Ile Met
   65 70 75 80
   His His Gln Arg Arg Arg Arg Ala Val Ala Val Ser Glu Val Glu Ser
   85 90 95
   Leu His Leu Arg Leu Lys Gly Ser Arg His Asp Phe His Val Asp Leu
   100 105 110
   Arg Thr Ser Ser Ser Leu Val Ala Pro Gly Phe Ile Val Gln Thr Leu
   115 120 125
   Gly Lys Thr Gly Thr Lys Ser Val Gln Thr Leu Pro Pro Glu Asp Phe
   130 135 140
   Cys Phe Tyr Gln Gly Ser Leu Arg Ser His Arg Asn Ser Ser Val Ala
   145 150 155 160
   Leu Ser Thr Cys Gln Gly Leu Ser Gly Met Ile Arg Thr Glu Glu Ala
   165 170 175
   Asp Tyr Phe Leu Arg Pro Leu Pro Ser His Leu Ser Trp Lys Leu Gly
   180 185 190
   Arg Ala Ala Gln Gly Ser Ser Pro Ser His Val Leu Tyr Lys Arg Ser
   195 200 205
   Thr Glu Pro His Ala Pro Gly Ala Ser Glu Val Leu Val Thr Ser Arg
   210 215 220
   Thr Trp Glu Leu Ala His Gln Pro Leu His Ser Ser Asp Leu Arg Leu
   225 230 235 240
   Gly Leu Pro Gln Lys Gln His Phe Cys Gly Arg Arg Lys Lys Tyr Met
                   245                 250                 255
   Pro Gln Pro Pro Lys Glu Asp Leu Phe Ile Leu Pro Asp Glu Tyr Lys
               260                 265                 270
   Ser Cys Leu Arg His Lys Arg Ser Leu Leu Arg Ser His Arg Asn Glu
           275                 280                 285
   Glu Leu Asn Val Glu Thr Leu Val Val Val Asp Lys Lys Met Met Gln
       290                 295                 300
   Asn His Gly His Glu Asn Ile Thr Thr Tyr Val Leu Thr Ile Leu Asn
   305                 310                 315                 320
   Met Val Ser Ala Leu Phe Lys Asp Gly Thr Ile Gly Gly Asn Ile Asn
                   325                 330                 335
   Ile Ala Ile Val Gly Leu Ile Leu Leu Glu Asp Glu Gln Pro Gly Leu
               340                 345                 350
   Val Ile Ser His His Ala Asp His Thr Leu Ser Ser Phe Cys Gln Trp
           355                 360                 365
   Gln Ser Gly Leu Met Gly Lys Asp Gly Thr Arg His Asp His Ala Ile
       370                 375                 380
   Leu Leu Thr Gly Leu Asp Ile Cys Ser Trp Lys Asn Glu Pro Cys Asp
   385                 390                 395                 400
   Thr Leu Gly Phe Ala Pro Ile Ser Gly Met Cys Ser Lys Tyr Arg Ser
                   405                 410                 415
   Cys Thr Ile Asn Glu Asp Thr Gly Leu Gly Leu Ala Phe Thr Ile Ala
               420                 425                 430
   His Glu Ser Gly His Asn Phe Gly Met Ile His Asp Gly Glu Gly Asn
           435                 440                 445
   Met Cys Lys Lys Ser Glu Gly Asn Ile Met Ser Pro Thr Leu Ala Gly
       450                 455                 460
   Arg Asn Gly Val Phe Ser Trp Ser Pro Cys Ser Arg Gln Tyr Leu His
   465                 470                 475                 480
   Lys Phe Leu Ser Thr Ala Gln Ala Ile Cys Leu Ala Asp Gln Pro Lys
                   485                 490                 495
   Pro Val Lys Glu Tyr Lys Tyr Pro Glu Lys Leu Pro Gly Glu Leu Tyr
               500                 505                 510
   Asp Ala Asn Thr Gln Cys Lys Trp Gln Phe Gly Glu Lys Ala Lys Leu
           515                 520                 525
   Cys Met Leu Asp Phe Lys Lys Asp Ile Cys Lys Ala Leu Trp Cys His
       530                 535                 540
   Arg Ile Gly Arg Lys Cys Glu Thr Lys Phe Met Pro Ala Ala Glu Gly
   545                 550                 555                 560
   Thr Ile Cys Gly His Asp Met Trp Cys Arg Gly Gly Gln Cys Val Lys
                   565                 570                 575
   Tyr Gly Asp Glu Gly Pro Lys Pro Thr His Gly His Trp Ser Asp Trp
               580                 585                 590
   Ser Ser Trp Ser Pro Cys Ser Arg Thr Cys Gly Gly Gly Val Ser His
           595                 600                 605
   Arg Ser Arg Leu Cys Thr Asn Pro Lys Pro Ser His Gly Gly Lys Phe
   610 615 620
   Cys Glu Gly Ser Thr Arg Thr Leu Lys Leu Cys Asn Ser Gln Lys Cys
   625 630 635 640
   Pro Arg Asp Ser Val Asp Phe Arg Ala Ala Gln Cys Ala Glu His Asn
   645 650 655
   Ser Arg Arg Phe Arg Gly Arg His Tyr Lys Trp Lys Pro Tyr Thr Gln
   660 665 670
   Val Glu Asp Gln Asp Leu Cys Lys Leu Tyr Cys Ile Ala Glu Gly Phe
   675 680 685
   Asp Phe Phe Phe Ser Leu Ser Asn Lys Val Lys Asp Gly Thr Pro Cys
   690 695 700
   Ser Glu Asp Ser Arg Asn Val Cys Ile Asp Gly Ile Cys Glu Arg Val
   705 710 715 720
   Gly Cys Asp Asn Val Leu Gly Ser Asp Ala Val Glu Asp Val Cys Gly
   725 730 735
   Val Cys Asn Gly Asn Asn Ser Ala Cys Thr Ile His Arg Gly Leu Tyr
   740 745 750
   Thr Lys His His His Thr Asn Gln Tyr Tyr His Met Val Thr Ile Pro
   755 760 765
   Ser Gly Ala Arg Ser Ile Arg Ile Tyr Glu Met Asn Val Ser Thr Ser
   770 775 780
   Tyr Ile Ser Val Arg Asn Ala Leu Arg Arg Tyr Tyr Leu Asn Gly His
   785 790 795 800
   Trp Thr Val Asp Trp Pro Gly Arg Tyr Lys Phe Ser Gly Thr Thr Phe
   805 810 815
   Asp Tyr Arg Arg Ser Tyr Asn Glu Pro Glu Asn Leu Ile Ala Thr Gly
   820 825 830
   Pro Thr Asn Glu Thr Leu Ile Val Glu Leu Leu Phe Gln Gly Arg Asn
   835 840 845
   Pro Gly Val Ala Trp Glu Tyr Ser Met Pro Arg Leu Gly Thr Glu Lys
   850 855 860
   Gln Pro Pro Ala Gln Pro Ser Tyr Thr Trp Ala Ile Val Arg Ser Glu
   865 870 875 880
   Cys Ser Val Ser Cys Gly Gly Gly Gln Met Thr Val Arg Glu Gly Cys
   885 890 895
   Tyr Arg Asp Leu Lys Phe Gln Val Asn Met Ser Phe Cys Asn Pro Lys
   900 905 910
   Thr Arg Pro Val Thr Gly Leu Val Pro Cys Lys Val Ser Ala Cys Pro
   915 920 925
   Pro Ser Trp Ser Val Gly Asn Trp Ser Ala Cys Ser Arg Thr Cys Gly
   930 935 940
   Gly Gly Ala Gln Ser Arg Pro Val Gln Cys Thr Arg Arg Val His Tyr
   945 950 955 960
   Asp Ser Glu Pro Val Pro Ala Ser Leu Cys Pro Gln Pro Ala Pro Ser
   965 970 975
   Ser Arg Gln Ala Cys Asn Ser Gln Ser Cys Pro Pro Ala Trp Ser Ala
   980 985 990
   Gly Pro Trp Ala Glu Cys Ser His Thr Cys Gly Lys Gly Trp Arg Lys
   995 1000 1005
   Arg Ala Val Ala Cys Lys Ser Thr Asn Pro Ser Ala Arg Ala Gln Leu
   1010 1015 1020
   Leu Pro Asp Ala Val Cys Thr Ser Glu Pro Lys Pro Arg Met His Glu
   1025 1030 1035 1040
   Ala Cys Leu Leu Gln Arg Cys His Lys Pro Lys Lys Leu Gln Trp Leu
   1045 1050 1055
   Val Ser Ala Trp Ser Gln Cys Ser Val Thr Cys Glu Arg Gly Thr Gln
   1060 1065 1070
   Lys Arg Phe Leu Lys Cys Ala Glu Lys Tyr Val Ser Gly Lys Tyr Arg
   1075 1080 1085
   Glu Leu Ala Ser Lys Lys Cys Ser His Leu Pro Lys Pro Ser Leu Glu
   1090 1095 1100
   Leu Glu Arg Ala Cys Ala Pro Leu Pro Cys Pro Arg His Pro Pro Phe
   1105 1110 1115 1120
   Ala Ala Ala Gly Pro Ser Arg Gly Ser Trp Phe Ala Ser Pro Trp Ser
   1125 1130 1135
   Gln Cys Thr Ala Ser Cys Gly Gly Gly Val Gln Thr Arg Ser Val Gln
   1140 1145 1150
   Cys Leu Ala Gly Gly Arg Pro Ala Ser Gly Cys Leu Leu His Gln Lys
   1155 1160 1165
   Pro Ser Ala Ser Leu Ala Cys Asn Thr His Phe Cys Pro Ile Ala Glu
   1170 1175 1180
   Lys Lys Asp Ala Phe Cys Lys Asp Tyr Phe His Trp Cys Tyr Leu Val
   1185 1190 1195 1200
   Pro Gln His Gly Met Cys Ser His Lys Phe Tyr Gly Lys Gln Cys Cys
   1205 1210 1215
   Lys Thr Cys Ser Lys Ser Asn Leu
   1220
<210> 3
   <211> 50
   <212> DNA
   <213> Artificial Sequence
<220>
   <223> Description of Artificial Sequence: an artificially synthesized linker sequence
<400> 3
   ctagcgcggc cgcaggatcc gactacaagg acgacgatga caaatgataa 50
<210> 4
   <211> 50
   <212> DNA
   <213> Artificial Sequence
<220>
   <223> Description of Artificial Sequence: an artificially synthesized linker sequence
<400> 4
   gatcttatca tttgtcatcg tcgtccttgt agtcggatcc tgcggccgcg 50
<210> 5
   <211> 34
   <212> DNA
   <213> Artificial Sequence
<220>
   <223> Description of Artificial Sequence: an artificially synthesized primer sequence
<400> 5
   ggactagtct agaagctggg taccagctgc tagc 34
<210> 6
   <211> 29
   <212> DNA
   <213> Artificial Sequence
<220>
   <223> Description of Artificial Sequence: an artificially synthesized primer sequence
<400> 6
   ggactagtgt cgaccggtca tggctgcgc 29
<210> 7
   <211> 38
   <212> DNA
   <213> Artificial Sequence
<220>
   <223> Description of Artificial Sequence: an artificially synthesized primer sequence
<400> 7
   ggactagtgc catgggaccc gcagcggcag cgcctggg 38
<210> 8
   <211> 40
   <212> DNA
   <213> Artificial Sequence
<220>
   <223> Description of Artificial Sequence: an artificially synthesized primer sequence
<400> 8
   gggcggccgc acccctgtga atcgtgcagg ctgagttatt 40
<210> 9
   <211> 41
   <212> DNA
   <213> Artificial Sequence
<220>
   <223> Description of Artificial Sequence: an artificially synthesized primer sequence
<400> 9
   ggactagtac catgaagccc cgcgcgcgcg gatggcgggg c 41
<210> 10
   <211> 30
   <212> DNA
   <213> Homo sapiens
<400> 10
   ccctgtggtc aacctcgtag gcagagacca 30
<210> 11
   <211> 27
   <212> DNA
   <213> Homo sapiens
<400> 11
   ggcagttcgg agagaaagcc aagctct 27
<210> 12
   <211> 40
   <212> DNA
   <213> Artificial Sequence
<220>
   <223> Description of Artificial Sequence: an artificially synthesized primer sequence
<400> 12
   gggcggccgc caagttggac ttagagcaag tcttgcagca 40
<210> 13
   <211> 41
   <212> DNA
   <213> Artificial Sequence
<220>
   <223> Description of Artificial Sequence: an artificially synthesized primer sequence
<400> 13
   tggccttcac cattgcccat cagtctggac acaactttgg c 41
<210> 14
   <211> 41
   <212> DNA
   <213> Artificial Sequence
<220>
   <223> Description of Artificial Sequence: an artificially synthesized primer sequence
<400> 14
   gccaaagttg tgtccagact gatgggcaat ggtgaaggcc a 41
<210> 15
   <211> 31
   <212> DNA
   <213> Homo sapiens
<400> 15
   caccttaagt agcttctgcc agtggcagtc t 31
<210> 16
   <211> 32
   <212> DNA
   <213> Homo sapiens
<400> 16 32
   acaaacatta cggctatcct ccgagcatgg ag 32
<210> 17
   <211> 23
   <212> DNA
   <213> Homo sapiens
<400> 17 23
   ttctaagcac cgctcaagct atc 23
<210> 18
   <211> 22
   <212> DNA
   <213> Homo sapiens
<400> 18
   gggccttcat caccatattt ca 22
<210> 19
   <211> 19
   <212> DNA
   <213> Homo sapiens
<400> 19
   ccatgccatc ctgcgtctg 19
<210> 20
   <211> 20
   <212> DNA
   <213> Homo sapiens
<400> 20
   aggggccgga ctcgtcatac 20
<210> 21
   <211> 11
   <212> PRT
   <213> Artificial Sequence
<220>
   <223> Description of Artificial Sequence: an amino acid sequence obtained by expression of a DNA containing a restriction enzyme NotI recognition nucleotide sequence and a nucleotide sequence encoding a FLAG tag amino acid sequence
<400> 21
   Ala Ala Ala Asp Tyr Lys Asp Asp Asp Asp Lys
   1 5 10
<210> 22
   <211> 5
   <212> PRT
   <213> Homo sapiens
<400> 22
   His Glu Ser Gly His
   1 5
<210> 23
   <211> 19
   <212> DNA
   <213> Rattus sp.
<400> 23
   agcctagctc ccgatccaa 19
<210> 24
   <211> 21
   <212> DNA
   <213> Rattus sp.
<400> 24
   ccaccaccag agtctccaca t 21
<210> 25
   <211> 15
   <212> DNA
   <213> Rattus sp.
<400> 25
   aagcaggcgg ccgag 15
<210> 26
   <211> 21
   <212> DNA
   <213> Rattus sp.
<400> 26
   atcaaaggtg gaagaatggg a 21